**Europäisches Patentamt**

**European Patent Office**

**Office européen des brevets**

(19)

(11) **EP 1 628 234 A1**

(12) # EUROPEAN PATENT APPLICATION

(43) Date of publication:
**22.02.2006 Bulletin 2006/08**

(51) Int Cl.:
*G06F 19/00* (2006.01)

(21) Application number: **04425416.7**

(22) Date of filing: **07.06.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicants:
- **Universita' Degli Studi di Milano-Bicocca**
  **20126 Milano (IT)**
- **Consorzio Milano Ricerche**
  **20131 Milano (IT)**

(72) Inventors:
- **Archetti Francesco**
  **20145 Milano (IT)**

- **Zimolo Edoardo Luigi**
  **20020 Solaro (MI) (IT)**
- **Roggia Luigi**
  **20052 Monza (IT)**
- **Fantucci Piercarlo**
  **28066 Galliate (NO) (IT)**
- **Gaiji Noura**
  **20147 Milano (IT)**

(74) Representative: **Cantaluppi, Stefano et al**
  **Cantaluppi & Partners S.r.l.**
  **Via Matteotti 26**
  **35137 Padova (IT)**

(54)  **Method of construction and selection of virtual libraries in combinatorial chemistry**

(57)  A method of construction and selection of virtual libraries in combinatorial chemistry is described, comprising the steps of: preparing a three-dimensional structure of a target macromolecule (PROT); determining one or more receptor sites of the macromolecular target (PROT); creating a first virtual library (300) of compounds starting with at least one input library (100;200); calculating a plurality of molecular descriptors for each molecule of the first virtual library (300), generating a second virtual library (400) containing each molecule of the first library (100; 200; 50) and the values of the molecular descriptors; selecting a representative subset (500) of the second virtual library (400); calculating for each molecule belonging to the representative subset a value of a quantity ($E_{dock}$) associated with the formation of a bond between the target macromolecule (PROT) and each molecule belonging to the representative subset (500); and obtaining by way of simulation through "Machine Learning" methods for each molecule of a plurality of molecules of the second virtual library (400) and not belonging to the representative subset (500) a value of the quantity ($E_{dock}$) associated with the formation of a bond between the macromolecular target (PROT) and each molecule of a plurality of molecules not belonging to the representative subset (500).

FIG. 1

EP 1 628 234 A1

**Description**

The present invention is related to a method of construction and selection of virtual libraries in combinatorial chemistry in accordance with the preamble of claim 1.

In many fields of chemistry, it is desirable to be able to synthesize a compound (whether it is a protein or a molecule) of distinct characteristics, for example pharmacological, and more in general functional or technological, connected with its stability, charge distribution and capacity to induce linear or non-linear responses such that it may be employed for a specific use. To such end, nevertheless, it is generally needed to synthesize and investigate the properties of many numerous compounds since there are no previously definable classes of compounds having the chosen characteristics (or are very rarely defined and with limited effectiveness). Therefore a lot of time and economic resources are invested in order to select from a very high number of possible candidate compounds, those that actually have characteristics such to merit systematic tests to verify all of their properties and applicability to very specific problems.

A typical example is that of pharmaceutical research, in which the research of new drugs has always been more quickly oriented toward technologies which consent the screening of a very large number of compounds. The axiom at the base of this approach is that the molecule with pharmacological properties must be able to interact with a molecular target (the receptor site) and that the probability of identifying a new drug increases with the number of compounds examined or with the capacity to define, beforehand, the necessary chemical-physical requirements.

The advent of robotics in combinatorial chemistry has permitted the use of an approach based on "brute force": since the principle theories which make a molecule a good drug are not substantially known (i.e. it is not possible to identify the characteristics of the drug beforehand), an exhaustive examination is undertaken of all "drug-like" molecules belonging to one or more chemical classes. This technique, known as High Throughput Screening (HTS), is extremely effective, at least regarding the location of the molecules capable of interacting with the receptor site, since it does not require previous knowledge and may also identify compounds whose effectiveness would not be predictable on the basis of present historical pharmacological knowledge. In the '80s, at the appearance of this new approach, the number of tests reached 10,000 units/year, a result of enormous impact for those times that represented a notable change of scale with respect to traditional chemical pharmaceutical research. Recent progress has ensured that today up to 100,000 units may be tested per day. Notwithstanding this enormous increase in the speed with which molecular libraries of great dimensions are subjected to screening, the frequency with which new pharmacological specialties are achieved is steadily decreasing, with the evident trend toward high of research and implementation costs of the final drug.

In recent years a critical review of the experimental HTS technique has begun, essentially for its very high cost (immobilizations in robotic systems and use of highly qualified personnel), in particular related to the reliability of the screening results. In addition, it must be underlined that very rarely the HTS results provide indications which permit the elaboration of general interpretive models.

Biological data processing has offered a solution for this situation. The HTS approach may be advantageously substituted by a Virtual HTS (VHTS) approach, in which many of the experimental operations executed in HTS are substituted by simulations employing advanced data processing methods in which many of the experimental operations executed in HTS are substituted by simulations which employ advanced data processing, statistic and molecular modelling methods. The advantages offered by a VHTS approach are briefly summarised as follows. To begin with, the number of molecules generated and analysed per day depends only on the computing power employed. The cost of investment in advanced computing platforms is at least one order less than that required by experimental HTS installations. Moreover, HTS technology base *in silico* combinatorial chemistry requires, in order for it to achieve screening efficiency, that all chemical reactions employed are simple and kinetically very fast, for all compounds which make up the library. The VHTS approach, on the other hand, is free of constraints related to the difficulty of chemical synthesis, which may be studied in detail and refined only on a number of final compounds, much smaller than the initial dimensions of the molecular library. The VHTS technique performed using of supercomputers permits an early selection of potentially interesting molecules over an extremely large number of cases, rapidly, at low cost, also supplying interpretive indications of general applicability.

Clearly, even the VHTS technology is not without limitations that, at the present state of the methodological knowledge of the diverse aspects, which constitute scientific structure, may be associated with the following points. The results reliability of numeric simulations of the chemical-physical characteristics of very complex molecular systems (target protein - ligand) has not yet reached high levels: results may not generally claim absolute quantitative value. Nevertheless, such results may be used as optimal qualitative indications; particularly when they may be employed for an extensive series of compounds, they permit the obtaining of very useful indications of general character. The availability of software having the complete integration characteristics for the entire VHTS process is very limited. In particular, the very few commercially-available software products available are characterised by their slow upgrade regarding new methods, and are not particularly adapted for use on intensive-computing platforms. Present commercially-distributed software products confront (though often in an incomplete manner) the key points of VHTS separately, such as i) generation of the virtual libraries, ii) chemical and functional characterisation of the molecules, iii) resolution of the Quantitative Structure Activity Relationship problem (QSAR. This is a mathematical model which correlates several molecular quantities with a physical-chemical - or biological -- property). In particular, generally missing from classic QSAR processing is the interaction energy parameter of each single molecule with a specific receptor site. Moreover, QSAR modelling is in general carried out with conventional statistical models of low effectiveness.

Description of the Invention

A first object of the method according to the invention is to generate a virtual library of compounds, each characterised by a plurality of "descriptors", or rather a plurality of values characterising its physical-chemical properties. The selection of a subset from the library, or rather a subset of "potentially interesting" molecules is therefore made by using Machine Learning methods.

These objects and still others highlighted below are achieved by the invention through a method of construction and selection of virtual libraries in combinatorial chemistry, made in accordance with the enclosed claims.

Brief description of the drawings

Further characteristics and advantages of the invention shall result clearer from the detailed description which follows of a preferred embodiment of the invention illustrated as a significant and non-limiting example, with reference to the enclosed drawings in which:

- Figure 1 is a block diagram of the method according to the invention;
- Figure 2 is a diagram of a step of the method according to the invention;
- Figure 3 is a block diagram of a step of the method of Figure 1;
- Figure 4 is a block diagram of a further step of the method of Figure 1;
- Figure 5 is a schematic representation of a step of the method according to the invention;
- Figure 6 is a block diagram of a further step of the method according to the invention;
- Figure 7 is a diagram of compounds employed in the method according to the invention;
- Figures from 8 to 13 are block diagrams of a further step of the method according to the invention.

Preferred Manner of Achievement of the Invention

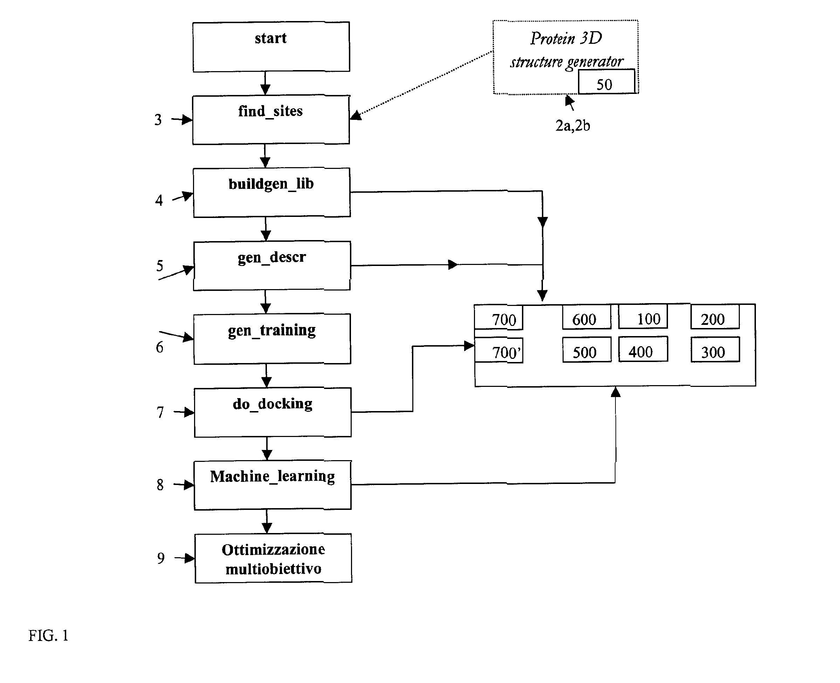

A block diagram of the method according to the invention is represented in Figure 1.

Such method proposes, starting from a library of compounds created by way of the same method, the generation of a sub-library containing only those compounds that most satisfy the chosen criteria. Such criteria are based on the particular values of particular descriptors.

For simplicity, the application of the method of the invention will be exemplified by anticipating its application in "Drug design", nevertheless the method is applicable to any other sector in which, beginning with a plurality of compounds, it is necessary to select a subclass optimising specific criteria and/or aspects of chemical, physical and technological type, or rather to carry out a first screening of potentially interesting compounds (where "interesting" depends on the type of anticipated application), starting with an initial plurality of compounds.

*3D Protein Structure*

In the typical case of pharmaceutical research, the drug is made starting from the protein target to which the drug must be bonded. The three-dimensional structure (3D) of this protein may be calculated directly through a step 1 of the method according to the invention as described below, or it may be supplied to the method of the invention as data acquired by external libraries 50 (as for example illustrated in Figure 1, where the protein structure is made from an external library and/or supplied by the user).

As is known, the 3D structure of a protein may arise from experimental solid state research with the diffraction of X-rays or in solution with Nuclear Magnetic Resonance (NMR). The information supplied from each of these two techniques is very often incomplete, due to the low resolution or disorder of the crystal in the case of X-rays or due to the inadequate relaxation times in the case of NMR. It is not therefore infrequent that for specific parts of the protein, the atomic coordinates supplied by experimental measures are characterised by precisions largely below the required limit in the successive steps of the method according to the invention.

Still more critical is the situation when the 3D structure is not experimentally known. In such case, the structure must be reconstructed by using parts or fragments of known structures of proteins that in several regions show sufficiently high levels of similarity with the protein under examination. The procedure, known in the art as *homology modelling,* leads to a first estimation of the 3D structure, to a limited extent to several parts of the protein, while the structure of the missing parts must be reconstructed *ab initio* based on a few indications of general character, connected for example with the particular nature of the amino acids involved, or rather on the indications from the structures known for fragmentations of sequences similar to that in examination.

According to step 1 of the method (optional) according to the invention, the structure of the target protein is made in two different ways (steps 2a and 2b), or rather

2a: the overall structure of the protein is generated through the completion of the structure supplied by sequence homology data, in the case in which the entire 3D structure is not experimentally known.

2b: the generated structure by sequence homology or by incomplete experimental data is refined such that it leads to a conformation of the protein as close as possible to that of absolute minimum energy.

In step 2a, a part of the structure is optimised (the connection fragments), considering the structure known for homology set. In particular, the orientation of the amino acid side chains that make up the connection fragments is optimised. Nevertheless, according to the method of the invention, the optimisation of the space of the atomic coordinates which may have dimensions on the order of $10^4$-$10^5$ is not executed, but the number of the problem variables is notably reduced, considering the side chains as molecular fragments which re-orient themselves in rigid manner, without modifying their internal structure, fixed on standard values. In this case, the problem contains a number of variables which is equal to the number of side chains and which coincides with the angle of rotation around the connection bond of each side chain to the protein backbone. In addition, only the variables that make reference to spatially-proximal side chains are physically (energetically) coupled variables and require therefore a simultaneous optimisation. The problem therefore may have overall dimensions equal to 50-100 and is easily factored in problems of dimensionality not greater than 5-10. The algorithm employed is of near-deterministic type and is based on the use of transformed target functions. Be $f(x)$ the current value of the target function, and $f(x)$ , the lowest minimum specific value and independent variables inside the vector x. The associated transformed function has the form:

$$\Phi^{\alpha}[f(\mathbf{x})] = \exp\left\{-\alpha[f(\mathbf{x}) - \bar{f}(\mathbf{x})]\right\}$$

with $\alpha$ parameter chosen such to "highlight" the absolute minimum and to render the hypersurface less "wrinkled". The function $\Phi$ is a monotonously rising function and its minimisation obtained with an iterated research of local minima of $f(x)$ leads to the overall solution. This optimisation methodology is effectively applied to problems with a limited number of variables (for example, less than 20) and for those in which the definition and evaluation of the first derivative (and eventually second) of $f(x)$ are relatively simple.

In step 2b, alternatively, the refinement of the overall structure of the protein is simplified and made more effective, exploiting two separate optimisations: the first in the space of the single torsional variables (dihedral angles or angles of rotation around single bonds), the second in the entire space of the degrees of freedom. The only degrees of torsional freedom for the whole protein are in high numbers, and the complexity of the problem does not allow the adoption, in general, of methods of direct overall optimisation. The overall optimisation approach which is employed mainly in this step 2b (overall optimisation of the protein structure, in the space of torsions only) is of stochastic type and is reproduced by the Simulated Annealing (SA) technique or by one its variants of Fast SA (FSA). The random sampling of the hypersurface may be from the beginning conducted with conventional techniques such as that of Monte Carlo (MC, MCFSA), or rather recurring to the sampling of the hypersurface produced by molecular dynamics trajectories (MD, MDFSA). For a relatively small number of variables, the most promising is the MCFSA method, which in the present step is such to simultaneously propagate a population of configurations, that is to produce at the same time a high number of independent Markov chains. Preferably, step 2b according to the invention is implemented on hardware platforms with extended parallelism. In particular, utilising the MCFSA technique, each single individual i ( $i=1,...n_l$) which coincides with a configuration inserted in the initial population and characterised by a set of variables $\{X_j\}$, is allowed to generate, in accordance with the Metropolis MC criteria, up to a predetermined maximum $max_c$ of new "accepted" configurations based on their specific values of the objective function $f(x_i)$. At the termination of this partial propagation, the entire population therefore includes $max_c.n_l$ individuals which are thus subjected to selection in order to restore the size of the population to $n_l$. The selection consists in the choice of the individual $n_l$ with better values of $f(x_i)$. Such choice is however compatible with the possibility of *hill climbing,* which is the characteristic aspect of MCSA.

Of course what described above is the case in which the application environment of the method according to the invention is the pharmacological one, in which the macromolecule with which an element of the virtual library interacts is, precisely, a protein. In the case in which the application environment is different, in the step of the invention the macromolecule target may be a polymer (natural or synthetic: mixtures of rubbers, technological polymers, fibre optics, etc.) of interest, with which the elements of the virtual library (explained more in detail below) are called to interact.

*Receptor Site Individualisation and Characterisation*

Once the three-dimensional structure of the protein is obtained, in the following the protein target is then called PROT (or in the case of a non-pharmaceutical application environment, simply macromolecule target), either through an external database or step 2a or 2b of the method according to the invention, in step 3 of the method, called in the following **find_sites,** receptor sites are located and characterised inside the target protein (the protein's three-dimensional structure and amino acid sequence is known from the previous step). Information related to each protein is preferably contained in a 3D structure file of format xxxx.pdb; nevertheless each format type is acceptable by the method according to the invention.

The localisation of the receptor sites is conducted by using computational geometry algorithms, which permit the localisation and therefore characterisation of cavities associated with the envelope surface of Van der Waals surfaces of the intersecting atomic spheres. These cavities are considered to be receptor sites on the condition that they are sufficiently large enough to host ligands with molecular complexity similar to those of compounds already recognised as drugs. The characterisation of the receptor site is instead conducted by mapping several characteristics of the residual amino acids on the inner surface of the cavity, which make up the environment of the cavity itself, and more in detail, of the atoms that compose such residue. Among the mapped characteristics, the following should be reminded:

- *The net atomic charges:* if greater than a predetermined threshold, for example this threshold is in the present method fixed equal to the value |0.2|, they identify zones of elevated hydrophilicity; on the other hand if less than |0.2| they define hydrophobic zones;
- *The atomic polarisability:* if high they indicate zones generally adapted for interaction with exogenous molecules;
- *Intermolecular hydrogen bonds (H-bonds):* the existence of H-bonds inside the receptor pocket is complementary to the characteristics of hydrophilicity of the pocket itself. The existence of numerous H-donor or H-acceptor atoms generally indicates that the zone is probably well adapted for interaction with the solvent, i.e. it presents a high accessibility for the solvent and therefore must in some manner be exposed on the outer surface of the protein.

At the conclusion of this calculation, techniques of computational geometry are applied by the invention method, and in particular algorithms based on Voronoi diagrams and on tetrahedralisation methods of the space surrounding the protein, and finally, to the definition of the associated $\alpha$-spheres. Once the cavity is identified, its dimensions and degree of exposure to the solvent (or rather its superficiality character) is determined by an algorithm that progressively fills the cavity with maximum packing spheres.

Once the cavity is identified, all of the items, which appear inside the cavity itself, are also identified. The envelope surface is therefore generated of the Van der Waals atomic surfaces of the atoms inside the cavity; applying colour scale techniques, the following properties are mapped on such surface:

- Atomic Contributions of Hydrophilicity;
- Atomic Contributions of logP , where P indicates the coefficient of octanolwater division;
- Electrostatic Potential, assumed to be generated by a positive and punctiform exploratory charge, on all points of the envelope surface.

The analysis of the properties mapped on the surface is of enormous importance because it permits the individualisation of general stereo-electronic characteristics with which the different ligands must comply to satisfy criteria of complementarity with the receptor site.

The receptor site research conducted by such method has characteristics of complete generality because it can highlight the existence of *all* possible receptor sites and not just those that present structural analogies with the receptors identified experimentally by X rays. The possible existence of additional receptor sites inside the same protein on the one hand increases the time of computing required by the analysis of the virtual libraries of molecules, as described below, but on the other hand may provide more abundant information than those directly accessible by experimental means.

*Creation of the First Virtual Library*

Once obtained the structure of the protein target and its receptor sites, the method according to the invention foresees a further step 4, denominated build_lib, in which a first virtual library 300 is generated beginning with several scaffolds (templates) and from a library of substituents. In other words in this step of the invention the user supplies a library of input, as described in detail below. Preferably a first and a second input library, 100 and 200 are provided, saved in a memory accessible by the present step of the method according to the invention.

The first virtual library 300 is constructed from base models, said scaffolds and indicated with $S_i$ (i = 1,......$n_s$) contained in the input library 100, which are just reference compounds, predetermined by the user, whose molecular structures concur to define the chemical classes of the compounds making up the library. The case of a single scaffold S is described here: the generalisation of $n_s > 1$ is obvious and reduces itself, at the coding level of the program and generation of corresponding library, to the repetition of the same block $n_s$ times.

For each of the scaffolds $S_i$ considered and contained in the first input library 100, the position and number $A_i$ of the connection points are defined ($A_i$, i=1,....$n_s$), i.e. the points of each scaffold where a bond with a particular substituent is possible. The substituents make part of the second input library 200 which comprises a plurality of R species ($R_j$, j=1,...,$n_R$), of opportune size and structure such to guarantee the molecular diversity of the compounds generated in combination with the scaffolds. The total number of the molecular species which may be generated $N_{mol}$ (also including all forms of symmetrical substitution) is equal to:

$$N_{mol} = \sum_{i=1}^{n_s} (n_R)^{A_i}$$

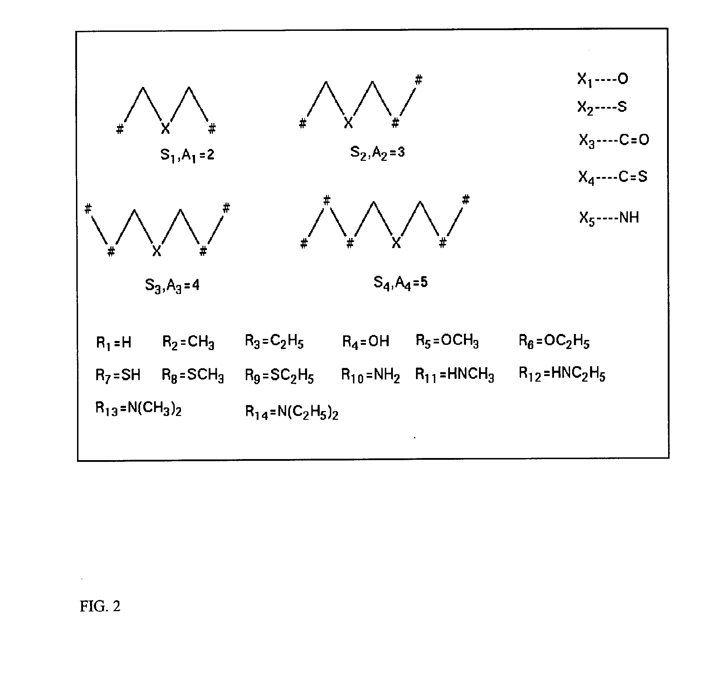

An example of molecular library composition is given in the diagram of Figure 2, from which it appears evident that the size of the library may be very large. For each scaffold, the point of connection of each residue is indicated with "#", an illustration of the residues employed is defined in the lower part of Figure 2.

In Figure 2, $S_i$ indicates the scaffolds; $X_i$ are substituents inserted in the scaffold in order to augment its structural flexibility; $R_i$ are substituents treated in a combinatorial manner.

It is easy to calculate the total number of the molecules in the following manner:

| Scaffold | $S_1$ | Number of Species | $14^2$ | = 196 |
|---|---|---|---|---|
| | $S_2$ | | $14^3$ | = 2744 |
| | $S_3$ | | $14^4$ | = 38416 |
| | $S_4$ | | $14^5$ | = 537824 |
| Total | | | | = 579180 |
| Total for all scaffolds | | | | = 2.316.720 |

The so formed library represents a list of "potential drugs".

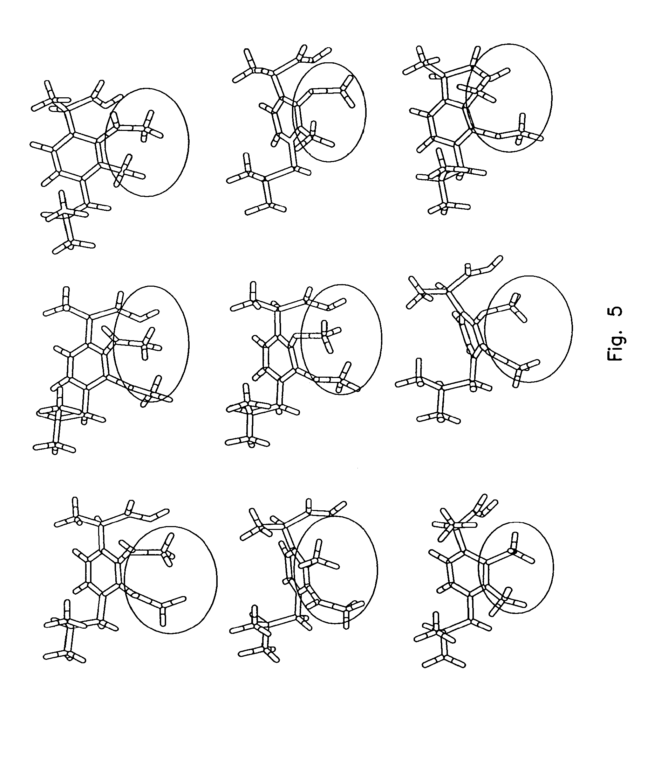

Figure 5 represents an example with a single scaffold, three substituents and two connection points: the substituents $NHCH_3$, $OCH_3$, $CH_3$ are bound to the scaffold at designated connection points (indicated in the figure).

The step **build_lib** 4 comprises the following main routines:

1. **build_lib2**
2. **read_scaff**
3. **read_sub**
4. **build_molec**
5. **out_lib**

**1) build_lib2**

**build_lib2** is essentially the control program.

**2) read_scaff**

The routine reads, from the first input library defined by the user, the composition and molecular geometry data of each scaffold S. One scaffold is defined by the following data, contained in the first library:

i) name_ of_ scaffold number_of_atoms.
*For each atom:*
ii) chemical_symbol; code (defined below); _x, _y, _z coordinate; net charge;

The format used is preferably a xxxx.pdb format, of which however only the above listed fields are considered. The use of the xxxx.pdb format in read_scaff (and in read_sub, see below) is motivated by the fact that such format is recognised by the totality of the 3D molecular structure visualizes.

**3) read_sub**

The routine reads (in loop), from the second input library defined by the user, the composition and molecular geometry data of each substituent R. A substituent is defined by the following data:

i) name_ of_ substituent number_ of atoms
For each *atom:*
ii) chemical_symbol; code; _x; _y; _z coordinate; net charge;
The format is the same of that used in **read_scaff.**

Sections 1) and 2) use the following service routines, essentially used in free format input processes:

record (separates into words the string of characters read from the input stream record)
inpa (converts a word into a string of characters and recognises its length)
inpi (converts a word into a integer value)
inpf (converts a word into a real value)

Regarding the 'code' data which appears in 1) and 2), this is a letter (A,B,C,.....) which identifies several hydrogen atoms in S and in R.
These hydrogen atoms, after deletion, identify the connection points on the scaffold or the connection point of a specific R. Clearly, a scaffold S or a substituent R may be characterised by several codes A,B,C,.... Several of these are selected in input by the user, in the case of S in order to define the effective number of connection points and in the case of R in order to define the orientation (contact point) of the substituent.
Here below the case of the substituent $CH_3$-CH(OH)-$CH_2$-$CH_3$ (sec-butyl alcohol) is described, which clarifies the general structure of a section of the second input library of substituents. The structure of the first input library of scaffolds is entirely analogous.

### CH₃-CH(OH)-CH₂-CH₃  15

| HETATM | 1 O | * | 1 | 0.597 | 1.474 | 0.578 | 0.00 | O |
|---|---|---|---|---|---|---|---|---|
| HETATM | 2 H | A | 1 | 1.181 | 2.123 | 0.148 | 0.00 | H |
| HETATM | 3 C | * | 1 | 0.332 | 0.438 | -0.365 | 0.00 | C |
| HETATM | 4 H | B | 1 | 1.290 | -0.010 | -0.653 | 0.00 | H |
| HETATM | 5 C | * | 1 | -0.335 | 1.048 | -1.592 | 0.00 | C |
| HETATM | 6 H | * | 1 | -0.554 | 0.287 | -2.347 | 0.00 | H |
| HETATM | 7 H | * | 1 | -1.268 | 1.554 | -1.322 | 0.00 | H |
| HETATM | 8 H | C | 1 | 0.312 | 1.807 | -2.044 | 0.00 | H |
| HETATM | 9 C | * | 1 | -0.564 | -0.618 | 0.287 | 0.00 | C |
| HETATM | 10 C | * | 1 | 0.107 | -1.293 | 1.475 | 0.00 | C |
| HETATM | 11 H | * | 1 | 0.335 | -0.574 | 2.268 | 0.00 | H |
| HETATM | 12 H | * | 1 | -0.551 | -2.058 | 1.897 | 0.00 | H |
| HETATM | 13 H | D | 1 | 1.041 | -1.779 | 1.175 | 0.00 | H |
| HETATM | 14 H | * | 1 | -1.493 | -0.155 | 0.638 | 0.00 | H |
| HETATM | 15 H | E | 1 | -0.836 | -1.383 | -0.449 | 0.00 | H |

END

In the example related to CH₃-CH(OH)-CH₂-CH₃, 5 different connection points are identified (A,....E).

The fields of the first/second library 100, 200 actually used in input by **read_scaff** and **read_sub** are the fields 3, 4, 6, 7, 8.

#### 4) build_molec

This is the essential routine of the build_lib step, which is logically composed by the sections described here below. For a better comprehension of the program structure, below the points inside S are denominated "substitution points" and the points inside each R "connection points".

4.1 For each specific scaffold S, the number and position of the substitution points in S are identified

4.2 The substituents R defined by the user are identified (type of substituent and predetermined connection point)

**4.3** Loop on the connection points in S

**4.4** Nested loops in numbers equal to the number of substituents $n_R$ (4.4.1, 4.4.2, ..., 4.4.$n_R$)

**4.5** For each connection point in S and for each substituent R, i.e. for the indices of control of the loops 4.3 and 4.4.1, 4.4.2, ..., 4.4.$n_R$. :

- Definition of the versor along the direction identified by the substitution point in S and its hydrogen atom to be removed.
- Removal of the hydrogen atom and shifting of the molecular connectivity data inside S.
- Removal of the hydrogen atom bound to the connection point in R and shifting of the molecular connectivity data inside R.

- Calculation of the distance between the substitution point in S and the connection point in R on the basis of their chemical and topological characteristics. This defines the effective coordinate in which the connection point of R is positioned and, along with it, all of R in a rigid manner.
- Modification of the torsion or rotation angle around the bond between the point of substitution (in S) and the point of connection (in R) in order to optimise the non-bond interactions.
- Closing of the loops-4.4.$n_R$, 4.4.$n_{R-1}$, .... 4.4.2, 4.4.1.
- Closing of the loop 4.3.

The molecule generated by S and by a particular combination of the substituent series R, is complete. It may be noted that in 4.5 the interaction of non-bonds between each substituent R and the remainder of each molecule in formation was optimised in cascade, precisely for each single R. This corresponds to the assumption that the spatial configuration of the molecule is not determined by the joint interactions of the diverse substituents. To overcome this limitation, proceed with step 4.6:

4.6 The orientation of all substituents is optimised with a process of Monte Carlo Simulated Annealing (MC-SA), which must guarantee an optimal approximation of the structure of minimum overall energy. The objective function to minimise in such case is represented by the sum of the contributions of type Van der Waals and of Coulombian type of the non-bond interactions. It is noted that, although for such method the orientation of the substituents is optimised in a rigid manner (i.e. without altering the inner structure with each substituent), the procedure permits a good sampling of the space of the torsional configurations.

The complete optimisation of the molecular structure is deferred to the **gen_descr** step, which represents the subsequent step of the method according to the invention, which is delegated to the generation of the optimal molecular structure of each molecule of the first virtual library 300 and to the calculation of the associated molecular descriptors.

For the execution of the **build_molec** tasks a series of service routines are employed, the main of which are:

**load_data:** load in memory a vast series of atomic data, necessary for the chemical characterisation of each atom inside the molecule and for the automatic determination of the connectivity matrix (i.e. the matrix which contains, for each atom i, the index of all atoms $j(i)$ directly tied to $i$) and of the matrix of the bond orders (i.e. the matrix which contains for each bond $i$-$j(i)$ the indication of single, double, triple or resonant bond).

**get_conn:** automatically generates the connectivity matrix and the matrix of the bond orders. On the basis of the connectivity and bond orders, the "chemically optimal" distance is determined between substitution point in S and contact point in R.

**gen_rot:** generates the matrix of 3D rotation, for arbitrary rotation angles around arbitrary axes of rotation. The axes of rotation are usually identified, in the case of examination, by an ordered couple of atoms, i.e. the axis of rotation is actually identified by the vector that points from the first to the second atom.

do_rot: executes the rotation by using the matrix defined in **gen_rot.** The strategy utilised employs a rotation matrix calculated only once and used for the rotation of an entire group of atoms.

In **gen_rot** and **do_rot** the number of floating-point operations is accurately minimised. In fact, the number of rotation operations to execute in the MC-SA process of overall optimisation of the orientation of all substituents may be on the order of $10^5$-$10^6$, which requires an accurately optimised code.

**gen_chg:** calculates the net charges with a method of equalisation of the effective atomic electronegativity. The method employed is rapidly convergent. The net atomic charges are necessary in the definition of the electrostatic contributions of the non-bond interactions.

It should be noted that step 4 build_lib foresees the existence of a first and a second input library 100, 200 respectively comprising the collection of scaffolds and substituents. The substituents, however, may be generated by the same step 4 **build_lib,** due to the fact that the structure of the files of scaffolds and files of substituents is entirely equivalent. Scaffolds and substituents may therefore be interchanged as desired.

One example of recursive construction of the substituent library is presented here below. It is presumed that the molecule $CH_4$ is available. This is employed both as scaffold and substituent (the removable proton is indicated with asterisk)

$$CH_3H^*+CH_3H^*=CH_3CH_3CH_2H^*CH_3+CH_3H^*=CH_3CH_2CH_3CH_3CH\ H^*CH_3+CH_3H^*=(CH_3)_3CH...$$

Once the desired series of aliphatic substituents is obtained, the next step may be, for example, the generation of corresponding alcohols, assuming that the $H_2O$ structure is available.

For example:

$$CH_3H^*+HOH^*=CH_3OHCH_2H^*CH_3+HOH^*=CH_3CH_2OH...$$

This distinctive feature of the structure of the reference files and **build_lib** mode of operation renders this module extremely flexible for any future expansion of the scaffold and substituent libraries.

Finally, it may be noted that the construction procedure of the first virtual library 300, here defined, is adapted for being parallelized. Indeed, it may be considered that the generation of a single species is within a series of nested loops as:

# substitution

points inside

the scaffold

```
                                    jnode=1

        do  is1=1, nsub
        do  is2= 1, nsub
        do  is3= 1, nsub
        .

        .

        .
                        jnode=jnode+1
                        if (mod (jnode, ndodes). eq. mymode() ) then
                        ….. (generation of molecule)



        endo
        endo
        endo
```

nodes ≡# of nodes (CPU) available
my node () ≡function of library that supplies the number of present node

**5) out_lib**

This is the section that generates (or updates) the first virtual library 300. In this step of the method of the invention, the LV contains, for each molecule, the following data:

i) name_of_molecole number_of_atoms
*For each atom*
ii) chemical_symbol; _x; _y; _z coordinate
the first virtual library 300 thus obtained is therefore saved in a memory accessible to the subsequent steps of the method of the invention.

Alternatively, the first virtual library may be supplied directly by the user, for example as input library, in case it is already available.

*Molecular Structure and Descriptors for Each Molecule of the First Virtual Library*

The above listed output data of the out_lib routine which constitutes the first library 300 is the minimum data required by the subsequent step 5, called gen_descr, for the generation of the molecular structure by quantum mechanical method and of associated molecular descriptors of each molecule belonging to the first virtual library 300. In this step 5, the 3D structure of the elements of the first virtual library is obtained through approximate calculations of quantum mechanical type (QM). In addition the QM and all those not QM molecular descriptors are also calculated.

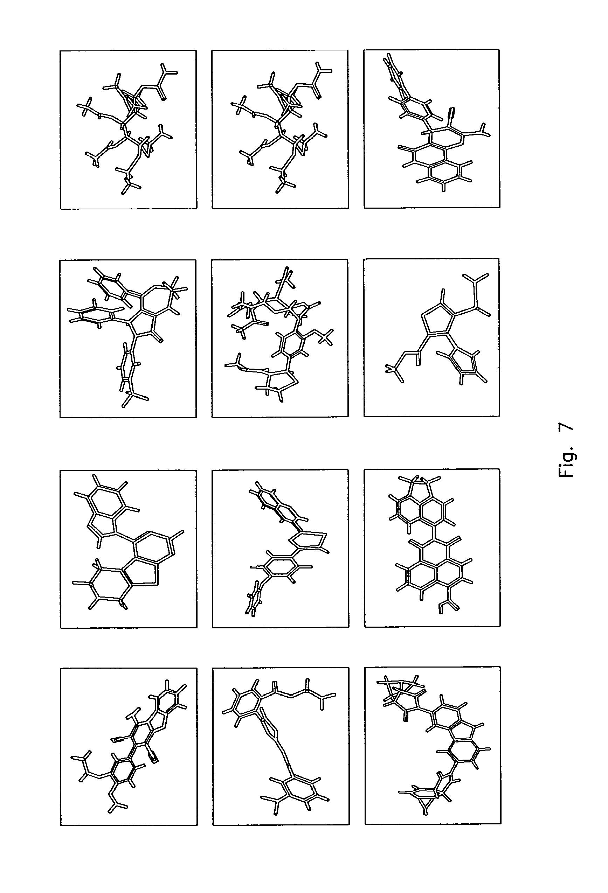

One example of typical molecular structure of compounds of the second library 400 is given in Figure 7.

The **gen_descr** step 5 comprises a great number of routines that may be classified in two groups. In the first group, there are the substeps designed for the optimisation of the 3D structures of the molecules which form the first virtual library (VL) and the calculation of several quantum mechanical (QM) molecular descriptors; in the second group there are the routines necessary for the evaluation of all non QM descriptors. From **gen_descr** step 5 a loop is executed on all the elements of the first virtual library 300 obtained in the preceding step 4 and the corresponding task is distributed to one of the available nodes. The parallelization obtained at this level requires a virtually null message-passing load between the nodes, and therefore permits the achievement of a very high efficiency.

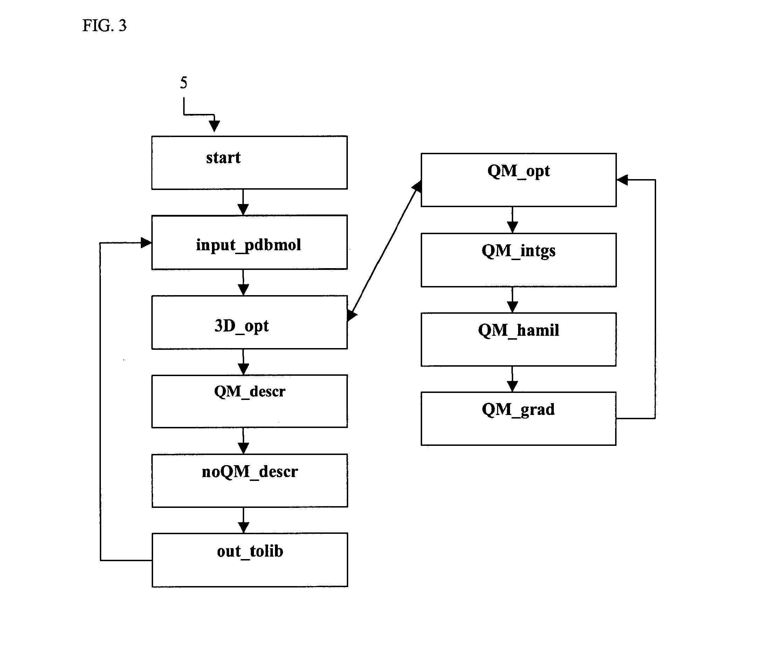

The routines comprised in **gen_descr** step 5 are listed in Figure 3 such that the logical interdependency is also evident.

**1) input_pdbmol**

The routine in object reads the chemical-structural data of a molecule in input, in xxxx.pdb format (see output of the gen_lib module) and contained in the first virtual library 300 generated in the preceding step 4. The molecular structure of each molecule of the first virtual library 300 is at this point optimised only regarding the degrees of torsional freedom of the rigidly inserted substituents on the scaffold, as said above. On the other hand, using an approximate QM approach optimises the valence structure.

**2) 3D_opt**

This is the module that runs the geometry optimisation. In turn it calls the following routines:

**2.1 QM_opt**
The routine changes - if necessary - the atomic coordinates of a molecule on the basis of energy gradients, obtained by a specific Hamiltonian model. **QM_opt** employs the steepest descending algorithms and conjugate gradients for coordinate updating. The first, as is known, is computationally simple but efficient only in zones far away from stationary points. The second algorithm is more reliable in proximity to the minimum of the objective function.
In the case of gradient components with very different magnitudes for different directions, an automatic selection algorithm is adopted which tends to favour movements along directions of higher gradient. From **QM_opt,** finally, it is controlled that the convergence criteria (gradient norm) are verified. If yes, the control is returned to **3D_opt,** otherwise the optimisation cycle proceeds. The updated atomic coordinates are transmitted to **QM_intgs.**

**2.2 QM_intgs**
The routine calculates all of the base integrals for a Hamiltonian model (essentially MNDO or AM1 which are two semi-empirical Hamiltonian molecular diagrams well adapted to small organic molecules), utilising algorithms optimised by a base of Cartesian Gaussians, centred on the nuclei. The atomic orbitals are expanded with four Gaussians whose exponents and contraction coefficients are *built-in* the program and held fixed. All monatomic quantities are precalculated, for atoms of type H, C, N, O, F, S, P, Cl, Br and I.

**2.3 QM_hamil**
The routine constructs the matrix of the Hamiltonian model and iteratively solves the problem of autoconsistency (SCF). At convergence, the matrix density is available which is transmitted to **QM_grad** for the calculation of the energy gradients.

**2.4 QM_grad**
The routine calculates the SCF energy with respect to the atomic coordinates. The gradient vector for a molecule containing $N_{at}$ atoms therefore is of $3 N_{at}$ size.

**3) QM_descr**

The routine calculates the molecular descriptors of QM nature for each molecule, whose structure is optimised as described above, i.e. all molecular quantities derivable from an approximate wave function, like those MNDO or AM1. QM molecular descriptors are the following:

1. Dissociation energy (atomisation) of the molecule
2. Ionisation potential
3. Dipole moment and its components
4. Quadrupole moment and its components
5. Net atomic charges

Refer to Table 1 for a complete list of descriptors (QM and non-QM).

**4) noQM_descr**

The routine calculates all molecular descriptors of non-quantum mechanical nature. The number of these descriptors may in reality be very high (over a thousand). Nevertheless, the number of these that is effectively calculated and memorised in the virtual library is equal to around 350. In the reduction of such number, the two following essential aspects are considered:

- An overly high number of descriptors generates the problem of redundancy of descriptive variables and imposes a great work of selection of variables (or combinations of variables) more reliable or more directly correlated with a target-property.
- Many of the descriptors that were introduced in literature in reality refer only to the atomic composition of the molecule, and do not even have direct connection with the typology of the molecule itself. This entire class of descriptors is held to be generally non-indicative of the stereo-electronic properties of the molecule under examination, and is therefore expressly ignored. In other words, those descriptors connected with the distribution of charge over the molecule are given wide priority, with its specific form and all its surface and volume characteristics.

**5) out_tolib**

The routine stores in a second virtual library 400, saved like the first library in a memory accessible to the subsequent steps of the invention method, all the information obtained for the molecule under examination. The output was preferably organised according to a format compatible with the *mysql* applications (an open source database). The *mysql* program may therefore directly access the second virtual library generated by **out_tolib,** requiring neither any format correction nor any intervention by the user.

Downstream from **out_tolib** the loop closes on all the elements of the first virtual library 300. It is noted that the order with which the molecules of the first virtual library are processed is not important. These in fact are inserted in the final second virtual library 400 with identifying labels. This means that during the processing of the first virtual library by the **gen_descr** step 5, each node of the parallel system may generate a sub-library of *mysql* type, in entirely independent manner from that operated by the other nodes. The final second library *mysql* may therefore be obtained with a single *global operation* at the end of the entire process.

The virtual libraries, generated according to the techniques described above and functionally characterised on the base of many molecular descriptors calculated by purely computational means, are of much wider use and interest than that strictly connected with the identification of lead pharmaceutical compounds. There are various problems, for example, connected with the environmental toxicity of several compounds, or rather the production of large classes of molecules to employ in situations in which the technological response of the product depends on the chemical nature of the same (additives for resins, rubbers, colorants, paints, composite products, etc.). Therefore what has been described up to now may be applied in any chemical environment in which a library of compounds must be generated, in this case virtual, quite large in order to test their characteristics.

*Identification* of *a Representative Subset from the Second Virtual Library*

Step 6, indicated with **gen_training,** is made up of routines called to solve the problem of identification of a representative subset of the entire virtual library 400, together with the descriptors, generated in the preceding step.

Before proceeding to the description of **gen_training** step 6, it must be made clear why it is necessary to supply a definition of the representative subset.

The conventional and experimental manner of conducting the screening of a class of compounds of pharmacological interest, such as those listed in the second virtual library 400, consists in the chemical synthesis of the elements of very large series of molecules, preferable "in silico" and the measure of the binding constants with proteins which act as molecular targets. Given the high number of compounds to analyse, the process is usually conducted with the use of highly robotised stations (High Troughput Screening, HTS). In the Virtual HTS approach, the experimental procedure is completely substituted by computational data processing, in which the chemical synthesis is substituted with the generation of virtual libraries of molecules, obtained with the steps of the method according to the above-described invention, and the measure of the binding constants is substituted by the calculation of the *docking* energy. By *docking* in this context it is intended the phenomenon of connecting a given compound (element of the virtual library) to a receptor site of an assigned protein (protein whose 3D structure, as anticipated in step 1 of the invention method, is supplied through an external library or directly calculated by the invention method). The calculation of the *docking* energy therefore consists of the simulation of the movement of the molecule being tested within the receptor cavity of the protein until the position of minimum energy, that is maximum interaction, is identified. The docking simulations were introduced in the VHTS trials for the projection of new drugs based on the postulate that the greater the docking energy the more the receptor site will be occupied in irreversible manner with a consequent conformational and therefore functional mutation of the protein itself. From this an intensified pharmacological activity of the ligand is obtained. Actually, the drug-receptor interaction is only one of the moments (even if a very important one) of the entire pharmakinetic process. It is true however that a strong interaction of the ligand with the receptor blocks in an almost irreversible manner the receptor site, impeding the access of other endogenous substrates, altering the protein's biochemical functionality; yet, a strong drug-receptor interaction may induce important, non-transitory conformational changes of the protein with a significant alteration of its biochemical behaviour.

Docking simulations may be from the beginning conducted at two different levels of accuracy modelling:

- *Rigid docking*
- *Flexible docking*

Rigid docking is certainly the more simple methodological approach: it is presumed in such a case that the protein and ligand (a molecule belonging to the second virtual library 400) maintain their internal structures unaltered during the bond, with the result that the optimum localisation and orientation of the ligand inside the cavity is a function of only 6 variables (3 Euler angles - or a quaternion - and three translation components). The reduced number of variables is well suited for confronting the problem in terms of *global optimisation* (GO), to solve by stochastic (general SA or FSA, genetic algorithms) or deterministic methods. The rigid docking technique will be based on the *maps of pre-calculated potential* approach, which permits a very efficient calculation of the atomic contributions to the total docking in terms of computing time.

The virtual libraries obtained through steps 2-5 of the method according to the invention and the screening of the properties of their components is a general methodological approach which may found, as already alluded, applications in non-pharmaceutical sectors. The method according to the invention is for example applicable to all problems of molecular type which entail discovering the magnitude of the characteristic response of a macromolecule, induced by the presence of a small molecule (outside the macromolecule, or exogenous) and by its specific interaction. In the pharmaceutical case the macromolecule is a protein and the response is the docking energy; in the case for example of superficial catalysis (oxides such as MgO, $SiO_2$, or catalysts supported on oxides) the macromolecule is identified with one (or few) solid support *slab* and the response is the energy of chemical adsorption; in the case of composite materials (mixtures, plastic materials, etc.) the macromolecule is invariably an oligomer or organic polymer, capable of interacting with a small molecule which acts as additive, and the system response is one of the polymer's chemical-physical or technological characteristics.

It is important to consider the following points (below reference will be made again to the pharmaceutical case):

- The number of the elements which compose the virtual library may even be very large ($5.10^5$ - $10^6$)
- The calculation of the docking energy for each single element of the library 400 on a receptor site of a protein composed by ~3000 atoms requires a relatively long time (for example not less than 200s on a single fast processor).
- The time of necessary calculation, even considering the possibility of using a multiprocessor system (with at least 20 processors) is extremely long (not less than 2-3 months).
- It is easy to predict that a considerable fraction of the elements of the second virtual library 400 is characterised by a very low docking energy, i.e. by an unsatisfactory affinity for the target protein.

The strategy utilised in the method according to the invention foresees the ability to simulate, at least for a part of the compounds of the second virtual library 400, the docking energy through machine learning techniques, attaining the individualisation of the elements of the virtual library 400 not suitable for this specific target (that is to be further tested as drugs), without having to recur to the explicit calculation of the docking energy and therefore considerably lowering the computing time.

Therefore step 6 of the method, known as **gen_training,** defines and optimises the composition of a representative subset 500 of the second library 400 (of dimensions equal to about 10% of the dimensions of the virtual library) on the basis of a parameter of *dissimilarity,* i.e. are included in the representative subset the most dissimilar molecules, such that the subset is maximally representative of the entire library. The docking energy of this representative subset in a subsequent step of the method according to the invention is calculated, while for the remaining molecules the energy is only estimated, as said above, always through a method step.

The dissimilarity is calculated with one of the following approaches:

1. Mode A- Dissimilarity based on molecular descriptors
2. Mode B- Dissimilarity based on linear combinations of the descriptors obtained from Principal Component Analysis (PCA).

3. Mode C- Dissimilarity based on linear combinations of descriptors, obtained minimising the covariance.

The definition of dissimilarity of a molecule $i$, $D_i$ which will be employed in the three above cited modes, is the following:

$$\Delta_i = \sum_{j \in S}(1 - \frac{\widetilde{\mathbf{d}}_\mathbf{i} \cdot \mathbf{d}_\mathbf{j}}{|\mathbf{d}_\mathbf{i}||\mathbf{d}_\mathbf{j}|}), \quad \widetilde{\mathbf{d}}_\mathbf{i} = (d_1, d_2, d_3, ....d_n)$$

where S represents the set (the virtual library 400 generated in the preceding steps), i and $j$ are elements of the set and $d_k$ is an original molecular descriptor (Mode A) or a linear combination from PCA (Mode B) or from minimisation of the covariance (Mode C).

The target function to be optimised is the sum of the individual dissimilarities:

$$\Delta = \sum_{i \in S}\Delta_i .$$

The procedure utilised in the case of Mode B (PCA) and Mode C should be mentioned.

First of all vectors $\mathbf{d}_i$ of the molecular descriptors are translated by using average values of the same descriptors

$$\mu_i = \frac{1}{N}\sum_{j=1}^{N}d_{ji} ;$$

the translation will be of the type $\mathbf{d'} = (d_1 - \mu_1, d_2 - \mu_2, ....d_n - \mu_n)$ .

The matrix $\mathbf{D} = (\mathbf{d'}_1, \mathbf{d'}_2, ...\mathbf{d'}_N)$ is therefore created, of n.N size, which originates the variance-covariance matrix $\mathbf{\Gamma} = \frac{1}{N}\mathbf{D}\widetilde{\mathbf{D}}$ of size n.n, which is diagonalised, according to:

$$\widetilde{\mathbf{V}}_\Gamma\mathbf{\Gamma}\mathbf{V}_\Gamma = \mathbf{\Lambda}_\Gamma .$$

In the case of Mode B, the eigenvalues, after ordering, are accumulated until they reach the fraction of 0.95 of the trace of $\Gamma$, which automatically selects the eigenvectors that account for 95% of variance. The PCA procedure is apparently costly (in the current example, the matrix D has dimensions of the type $(10^2 \cdot 10^5)$ and thus its construction is particularly onerous) but offers the great advantage of being able to reduce the space of the parameters (descriptors) in a very considerable measure. Many examples conducted with the set of 346 descriptors listed above have demonstrated that 95% of the variance may be accounted for by no more than 20 transformed variables.

Downstream of the PCA selection, the transformed parameters are obtained $\delta_i = \mathbf{V}_\Gamma\mathbf{d}_i$ .

Clearly appears that the number of scalar products of the type di $\cdot$ $\mathbf{d}_j$ (see the definition of dissimilarity) that must be calculated is extremely high. The reduction of dimensionality from 346 to ~20, and therefore the calculation of the scalar products $\delta_i \cdot \delta_j$, accounts for the enormous reduction of times.

In the case of Mode C, the matrix $\mathbf{\Gamma} = \frac{1}{N}\mathbf{D}\widetilde{\mathbf{D}}$ is diagonalised and from this the matrix $\Gamma^{-1/2} = \mathbf{V}_\Gamma\Lambda^{-1/2}$ is determined. The transformation of the variables (original descriptors) according to $\delta_i = \Gamma^{-1/2}\mathbf{d}_i$, corresponds with the definition of a variance-covariance matrix identity: $\Gamma^{-1/2}\Gamma \Gamma^{-1/2} = \mathbf{I}$.

The program therefore proceeds with the extraction of the representative subset, utilising a Monte Carlo-Simulated Annealing (MCSA) method of conventional type.

Said $v$ the size of the representative subset S, the algorithm goes on with the construction of a random collection of *v* molecules extracted from the entire subset of N molecules. The value

$$\Delta \; = \sum_{i \in S}^{v} \Delta_i$$

is calculated and thus the generation of the Markov chain proceeds, substituting at random a molecule inside S with a molecule outside S. The so modified set will be characterised by a different value of $\Delta$, and the substitution will be accepted with the Metropolis criteria, usually employed in MCSA. The MCSA procedure is prolonged until an overall temperature is attained below a predetermined threshold.

With the goal of obtaining more reliable results, once the vectors $\mathbf{d}_i$ or $\delta_i$, are organised in the mass memory, these are distributed on all available processors and different MCSA simulations are conducted in parallel. Of these, that considered to be the final solution is characterised by a higher total dissimilarity index value. The representative subset 500 of the second virtual library 400 is therefore calculated, for which the docking energy is also effectively calculated.

*Docking Energy Determination*

Step 7, identified with the name **do_docking,** is constituted by numerous routines that determine the docking energy of a particular molecule (element of the representative subset 500 calculated in step 6) on a particular receptor site. By "docking" in this context, as already outlined above, it is intended the connection of a given compound with a receptor site of the assigned protein, or the target protein of step 1. The calculation of docking energy therefore consists in the simulation of the molecule being tested within the receptor cavity of the protein until the position of minimum energy is identified, i.e. that of maximum interaction.

The approach employed by the method according to the invention draws on the pre-calculation technique of the maps of potential above a rectangular grid of cubic symmetry. The number of grid points is easily calculated: assuming that the entire dimensions of the cube containing the protein pocket considered as receptor site are (20x20x20) A and the pitch of the grid is 0.25 A, the total number of points is equal to 512,000. The number of grids to be calculated is variable and depends both on the general chemical composition of the ligand library and on the sophistication of the interaction potential desired for use.

The minimal form of the potential utilised in this step 7 of the invention method is of the type:

$$E_{\text{int}} \; = \; \sum_{i \in A, j \in B} \left\{ E_{ij}^{vdw} + E_{ij}^{Coul} + E_{ij}^{solv} \right\},$$

where A and B respectively indicate the ligand and the protein; the indices vdw, Coul and *solv* respectively indicate the non-bond contributions (van der Waals), those electrostatic and those of solvation.

It is to be noted that the term *vdw* is in reality factorised in a sum of terms, each of which corresponding to a specific, different *atom type* for each atom i. For organic molecules like the most common ligands, the foreseen types of atoms are: H, C (C non-aromatic, C aromatic) , N, O, F, P, S, Cl, Br and I, which signifies that the total number of the maps of potential to calculate is equal to 13.

Since the calculation of each of the maps is completely independent, this part of the **do_docking** task may be easily distributed by a series of different processors.

In each case, the calculation of the maps of potential is not the *rate-determining step* of **do_docking,** since such calculation must be executed only once.

The most burdensome task from the computational standpoint of **do_docking** is in its use for the determination of the docking energy of each single component of the representative subset 500. The problem is formulated in the following manner:

$$E_{dock} \; \min\{\Phi[t, \; \vartheta(s)]\}$$

$$t{=}(t_x, t_y, t_z)^T$$
$$s{=} (s_x, \, s_y, \, s_z)^T, \; |s|{=}1$$

*t* and *s* are respectively the translation vector and the versor around which an arbitrary rotation 9 occurs. The function

$E_{dock}$ must be optimised in a global manner.

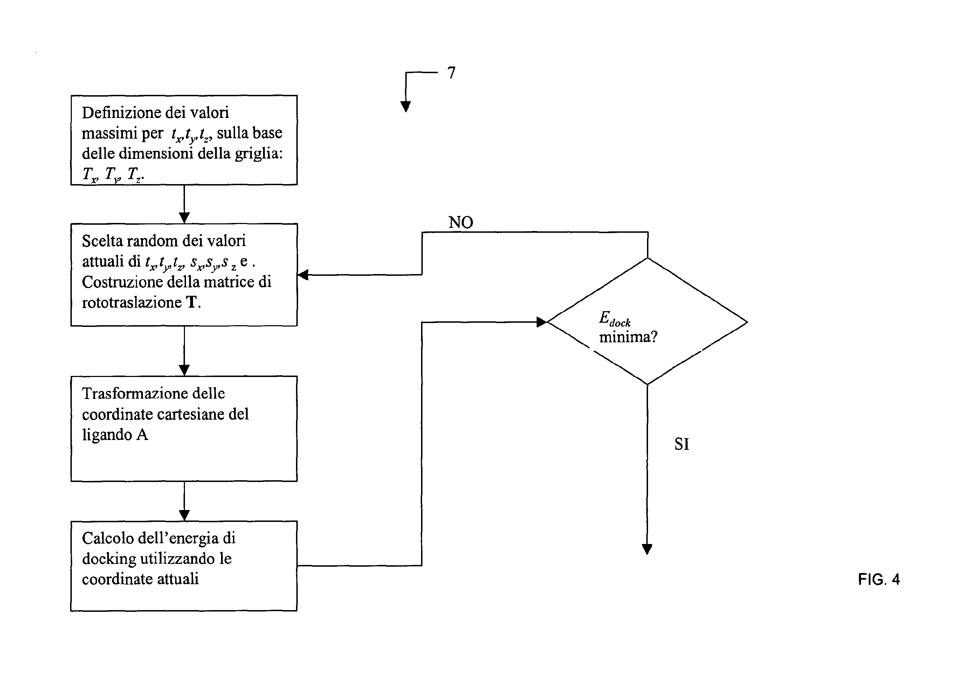

The algorithm therefore proceeds with the following steps, exemplified in Fig. 4:

- The maximum values of $t_x, t_y, t_z$ identified with $T_x, T_y, T_z$ are calculated on the basis of the grid dimensions.
- Values for $(t_x, t_y, t_z, S_x, S_y, S_z$ and $\vartheta)$ are therefore chosen in a random manner. The rototranslation matrix T is therefore constructed.
- The atomic coordinates of the atoms making up the ligand are transformed:

$$x'_A = Tx_A$$

- The docking energy is therefore calculated utilising the present coordinates $(x'_A, x_B)$.
- If the docking energy is not the minimum, the calculation is repeated by choosing in a random manner new values for $(t_x, t_y, t_z, s_x, s_y, s_z$ and $\vartheta)$.

The **do_docking** step foresees three different techniques of global optimisation of $E_{dock}$

1. Fast Simulated Annealing (FSA)
2. Genetic Algorithms
3. Direct Global Optimization

Methods 1 and 2 are of conventional type, while method 3 employs deterministic algorithms that guarantee the attainment of the global minimum of the function in a finite number of steps. The applicability of such algorithms is rendered possible by the fact that the problem of rigid docking is reduced in reality to a problem with a reduced number of variables $(t_x, t_y, t_z, S_x, S_y, S_z$ and $\vartheta)$.

The high computing time required by **do_docking** for each single element of the representative subset is essentially due to:

1. The number of times that the objective function $E_{dock}$ must be calculated is in general very high.
2. Each calculation of $E_{dock}$ requires the accumulation of a number of contributions ($ij$ couples, $i \in A$, $j \in B$) on the order of $10^5$.
3. Each contribution arises from a cubic interpolation, which utilises the eight values of the energy of a specific map, evaluated at the vertex of the elementary cube of the grid that contains the atom i in question.
4. The summed contributions on the $ij$ couples must be in addition summed by (at least) three terms that compose the potential.

The time of computing is maximally reduced, recurring to the optimisation of the algorithm of cubic interpolation and evidently to the parallelization which distributes the calculation relative to each element of the virtual library.

At the end of step 7 of the invention, therefore, the docking energy is calculated for each molecule of the representative subset 500. The whole set of molecules of the representative subset, their associated docking energy and their molecular descriptors forms the training set 600.

*Machine Learning Step*

From the preceding step, therefore, a training set 600 is created comprising all of the molecules of the representative subset 500, their molecular descriptors and their docking energy. According to a further step 8 of the invention method, an estimate is made with regards to the docking energy of the remaining molecules (the most part) of the second virtual library 400. To such end a machine learning approach is utilised by the method according to the invention.

Although in the following only the term "docking energy" is utilised, this step of the method, as in the preceding steps, may be applied not only to the pharmaceutical sector, but also to values of a quantity associated with the formation of a bond between the target macromolecule and a molecule of the representative subset.

The action strategy and recourse to *machine learning* (or expert systems) methodologies is based on the following points:

- The elements of the second virtual library 400 are subdivided into two groups, the first (group A) composed by about 10-20% of the elements chosen with techniques that guarantee their statistical representativeness in the entire library, that is the representative subset 500 calculated in step 6 of the invention method, and the second (group B) formed by the remaining elements. It is presumed that for all elements (groups A and B) molecular descriptors are already available.

• The docking energies, calculated in direct manner as described in the preceding step 7, are available just for the elements of group A.

Docking energies (usually negative, to indicate the attractive interaction) are comprised between the optimum values ($E_L$) (e.g. -10 Kcal.mol$^{-1}$) and poor values ($E_U$) (e.g. 0 Kcal.mol$^{-1}$). The basic idea is to train and validate a "computational" machine on the elements of group A in order to utilise it on the group B molecules, attaining the isolation in this manner of a molecule subset with higher affinities for the receptor site under examination. To build this, in step 8 of the method according to the invention, three fundamental methodologies of Machine Learning are referred to: Neural Network, Naïve Bayes classifier and Tree-Augmented Naïve Bayes classifier, in which it is foreseen, preceded by an adequate pre-processing sequence, the subdivision of the set A into K subsets, K-1 of which will be utilised in the construction of a forecast/classification model, while the remainder shall be utilised as a set of tests (in the case of the neural networks K is equal to 2).

At this point the methodology which presents the best results on the set of test may be employed for the prediction of the approximate value of the docking energy or of the class to which the group B molecules belong as a function of the docking energy.

Step 8 is articulated in the following substeps:

- **Pre-processing:** the data contained in the training set 600 which belong to group A are properly processed together with that of group B in order to obtain a new set through which forecast/classification models of the docking energy are constructed;

- **Construction of the models:** the data set obtained by the pre-processing subset are used to identify the performance limits of a Neural model, of a model of Naïve Bayes type and of a model of Tree-Augmented Naïve Bayes type;

- **Choice of the model:** models obtained by the preceding step are compared, in order to choose the model which presents least errors of forecast/classification;

- **Reconstruction of the chosen model:** the model identified by the preceding step is regenerated by utilising the molecules of group A;

- **Obtaining the docking energy:** forecast/classification of the docking energy of the molecules of group B, utilising the model produced by the preceding substep.

### 1) Pre-processing

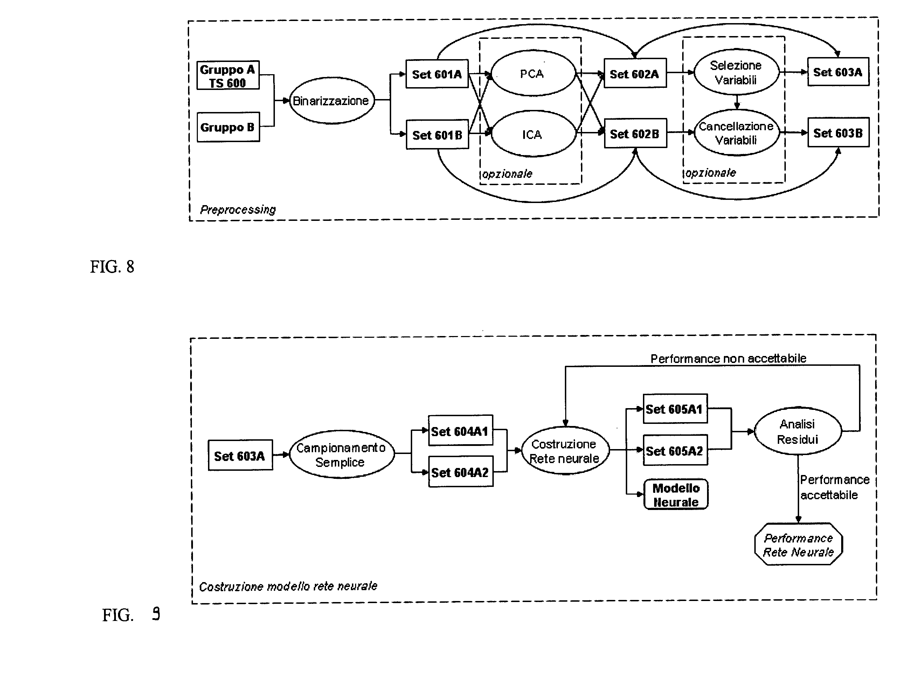

Figure 8 shows how the pre-processing cycle of the molecules that compose groups A and B is structured.

The pre-processing substep is constituted by the following operations:

- Binarization,
- Principal Component Analysis (PCA) or Independent Component Analysis (ICA) - optional operation,
- Variable Selection and Cancellation - optional operation.

### 1.1 Binarization Operation

Input data

Training set 600 constituted by the group A molecules and group B molecules, identified as a single data set.

The data contained in the training set 600 making up part of group A are structured in a table of m rows and n columns, in which the rows represent the molecules and the columns the variables which characterise each molecule (molecular descriptors and calculated docking energy).

Group B data is organised in a table with m rows and n-1 columns, in which the rows represent the molecules and the columns the molecular descriptors.

Function

The molecular descriptors may be of two types:

o Continuous, the molecules for the given descriptor may assume any real value within a given range (for example between -2.354 and +5.23, comprising both ends),
o nominal, the molecules for the given descriptor may assume any one value between those admissible, usually limited and finite in number (for example only the values 0, 4, 7, 8, and 10 or aa, cc, dd, kk).

The docking energy is as already said of continuous type and may assume values comprised in a specific range, for example between -10 and 0 Kcal.mol$^{-1}$.

The first operation done in the pre-processing step is that of adapting the molecules of the groups A and B such that the variables which describe them (the descriptors) are of continuous type. The binarization operation substitutes all descriptors of nominal type with corresponding variables of continuous type, transforming each of the descriptors into k new variables, where k are the values that the descriptor itself may assume. The new variables take on values of 0 or 1 for each molecule. For example, assuming that the molecules of group A and B have the values aa, *bk* and cc for the "descriptor $\alpha$" and that the first molecule has aa value, the second value *bk*, the remaining values cc, cc, cc, *bk, bk,* aa, to the end of the binarization, three new columns "$\alpha$_aa", "$\alpha$_bk", "$\alpha$_cc" will be added to the set with values $[1,0,0,0,0,0,0,1]^T$ for the variable "$\alpha$_aa" , $[0,1,0,0,0,1,1,0]^T$ for the variable "$\alpha$_bk" and $[0,0,1,1,1,0,0,0]^T$ for the variable "$\alpha$_cc". The names of the columns created are obtained by adding the approximate nominal value that the column represents to the name of the descriptor in question.

Output data

Set 601A constituted by the molecules of group A, 601 B constituted by the molecules of group B.
Sets 601A and 601 B are a transformation of input data, with equal numbers of molecules and made by the following new descriptors:

• descriptors of the input dataset of continuous type,
• variables with possible values for each molecule 0 and 1, derived from the binarization operation, which substitute the descriptors of nominal type.

**1.2 PCA - ICA Operation**

Input data

Set 601A and Set 601 B, treated like a single data set.

Function

The Principal Component Analysis (PCA) or Independent Component Analysis (ICA) operation is an optional operation of the machine-learning step. In other words, the machine-learning step may require a PCA or ICA step or neither of the two.

Principal Component Analysis (PCA)

The principal components are linear combinations between the variables: the first principal component collects the greater share of variance; the second (not correlated with the first) collects the greater share of the remaining variance and so on... The analysis of the principal components is in turn a factorial analysis; indeed it produces a set of principal components that may be considered new variables.
In the machine learning step, the use of the principal components permits, beginning with the sets 601A and 601B taken together and without considering the variable docking energy, the achievement of a new set of data built by new variables, precisely the principal components, which substitute the preceding ones. Generically a reduced number of principal components contains most of the information of sets 601 A and 601 B, so that the PCA also produces a reduction in the space to be analysed.

Independent Component Analysis (ICA)

The analysis of the independent component is a statistical technique for the decomposition of a set of complex data into its independent subparts. Thus, while the variables generated by the PCA method, taken two at a time, have zero correlation, the variables obtained by the ICA technique are statistically independent. In addition, the ICA may produce a reduction in the space to be analysed, but may leave it unaltered or even increase it.
In the machine learning step, the use of the principal components permits, beginning with the sets 601A e 601 B taken together and without considering the variable docking energy, the achievement of a new set of data built by new, non-ordered variables, precisely the independent components, which substitute the preceding ones.

Output data

Set 602A, Set 602B. The number of the molecules is equal to the corresponding input sets, while the new descriptors which represent them depend on the technique applied, i.e. there are three possible alternatives:

- PCA, set 602A is built from the principal components and from the docking energy, set 602B is composed just by the principal components. The number of principal components is equal to the number of variables of the input database (descriptors + binarised variables) and for each of these the share of the variance, which the component itself represents, is given as a percentage.
- ICA, set 602A is built from the independent components and from the docking energy; the set 602B is composed by the single independent components. The number of the independent components is subordinate to the formulation of the ICA algorithm.
- No technique applied, set 602A is equal to set 601A, set 602B is identical to set 601B.

## 1.3 Variable Selection Operation - Variable Cancellation

Input data

Set 601 A and Set 601 B, if the PCA or ICA operation is not executed.
Set 602A and Set 602B, if the PCA or ICA operation is carried out.

Function

The variable selection function and the consequent cancellation depend on the characteristics of the input data, i.e. if a PCA is carried out, or an ICA, or neither of the two. Also this operation, as the preceding one, is optional.
If the input setsd are sets 601A and 601B, the selection operation of the variables on set 601A is executed by means of a linear regression having the continuous descriptors and the variables generated by the binarization as input variables, and the docking energy as output variable. Once the regression is executed, a Test F is executed and the input variables having a value of test F smaller or equal to a certain threshold, fixed by default in this preferred example as equal to 0.05, are maintained in the process. The variables eliminated by the set 601A are then taken out also from set 601 B by way of the Variable Cancellation operation, so that the molecules of group A and B are represented by the same variables (descriptors and columns obtained with the binarization).
If the input sets are sets 602A and 602B and an ICA was executed, the variable selection operation is the same as that described above.
If the whole input are sets 602A and 602B and a PCA was executed, the variable selection operation is essentially the individualisation of the first principal Z components, where Z is chosen as a function of the variance represented by the same components (usually greater than 90% of the total). The cancellation operation on the set 602B maintains the principal components that were not removed from set 602A.

Output data

Set 603A, Set 603B. Their information content, exhibited through of a number of molecules equal to the input sets, depends on the process flux executed upstream. In particular:

- No PCA, ICA, and Variable Selection/Cancellation operation is applied. Set 603A is equal to set 601A and set 603B is equal to set 601 B.
- PCA operation executed. Set 603A contains the selected principal components and the docking energy, set 603B contains the chosen single principal components.
- PCA operation executed and no Variable Selection/Cancellation operation executed. Set 603A is equal to set 602A and set 603B is equal to set 602B.
- ICA operation executed. Set 603A contains the selected independent components and the docking energy; the set 603B contains the chosen single independent components. ICA executed and no Variable Selection/Cancellation operation carried out. Set 603A is equal to set 602A and set 603B is equal to set 602B.

## 2) Model construction

The model construction substep is built by the construction operations of a neural network model and by the construction of two Bayesian classifiers according to the Naïve Bayes and Tree-Augmented Naive Bayes techniques.

**2.1 Neural network construction**

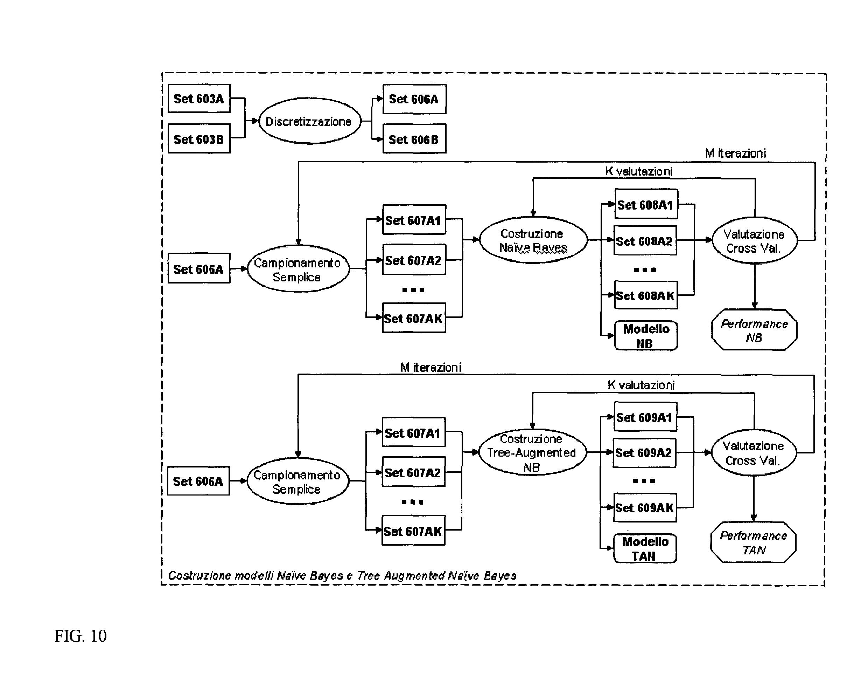

Figure 9 shows how the construction process of the neural model is structured by using part of the molecules of group A for the training step, the remainder for the test step.

The construction of the model of the neural network is carried out through the following operations:

- Simple sampling,
- Neural network construction,
- Residue analysis.

The cycle of construction of the network is iterated until the model performance is judged to be acceptable. At each cycle the architecture is modified in order to obtain improved docking energy forecasts.

**2.1.1 Simple sampling**

Input data

Set 603A.

Function

The simple sampling executes a random, uniform selection of the molecules contained in the input dataset, generating two new datasets: the first for training the neural network (learning set), the second for verifying the performance (test set).

The percentage of the molecules in the learning set is set by default to 80% of the total molecules contained in set 603A.

Output data

Sets 604A1 and 604A2, which from the columns (variables) standpoint present no modifications, but which are a subdivision of the molecules of the input set into two subsets, the first for neural network learning, the second for the model performance test.

**2.1.2 Neural network construction**

Input data

Sets 604A1 and 604A2.

Function

Set 604A1 is utilised for the construction of a neural network of feed forward type, where the input nodes are descriptors generated by the pre-processing process (descriptors of continuous type and variables deriving from the binarization process, either principal or independent components), the output node is the docking energy.

The network architecture is variable and by default it is structured with two hidden neuron levels, each composed of 8 or 16 nodes. Consequently the input nodes are all connected with the first level of hidden neurons, which are themselves all connected with the second level of hidden neurons, each of this finally connected to the output node.

The activation function is set, by default, equal to the hyperbolic tangent.

Set 604A2 is used to verify the predictions of the network on a set that was never used in the training step (hold out).

Output data

Set 605A1 is equal to set 604A1 with the addition of the column of the forecast docking energy computed by the model.

Set 605A2 is equal to set 604A2 with the addition of the column of the forecast docking energy computed by the model.

The Neural Model contains architecture and quantitative valorisation of the constructed neural network.

### 2.1.3 Residue analysis

Input data

Set 605A1, set 605A2.

Function

For each molecule sets 605A1 and 605A2 contain the docking energy value, calculated with the do_docking (Ed_C) step 7 process and the predicted docking energy from the constructed neural network model. The residue analysis is the mean to verify the effectiveness of the constructed model, through the following qualitative/quantitative measures:

- Scatterplot Ed_C versus Ed_P,
- Scatterplot residues (Ed_R) versus Ed_C. The residues are equal to (Ed_C - Ed_P),
- Histogram of the residues,
- Normality test of the residues (Shapiro-Wilk or KSL)

The analysis is separately executed on the learning set and the test set. If the results are not satisfying, the neural network construction process is repeated, modifying, based on the experience, the architecture of the network itself. If the results are instead deemed to be exhaustive, the network construction process is stated to be concluded.

Output data

Performance data of the neural network, which are highlighted through the scatter plots, the histogram of the residues and the normality test on the abovementioned learning and test sets.

### 2.2 Bayesian model construction

Figure 10 shows the structure of the construction process of the Naïve Bayes or Tree-Augmented Naïve Bayes model, using the k-folds Cross-Validation technique.
The construction of the two models is carried out through the following operations:

- Discretization,
- K-Folds Cross Validation, constituted in turn by the steps of:

  o Simple sampling,
  o Model construction (Naive Bayes or Tree-Augmented Naïve Bayes),
  o Cross Validation evaluation

### 2.2.1 Discretization

Input data

Set 603A and set 603B, containing the variables which describe each molecule (descriptors of continuous type and variables deriving from the binarization process, or principal or independent components). Set 603A also comprises the docking energy calculated with the simulation process.

Function

The Bayesian type classifiers require variable input of nominal type. The goal of this operation is to transform the continuous variables into corresponding discrete variables.
The docking energy of set 603A is subdivided into M classes; by default in this preferred example M is set equal to two, the first class identifying energy values of less than or equal to -6.5, the second docking energy greater than the chosen threshold.
The discretization of the remaining continuous variables (typically the descriptors or the principal or independent components) of the set 603A may be carried out in three ways:

- Applying the Fayyad-Irani technique, which executes a discretization based on entropy measures and dependent on the values taken on by the class, in the specific case of the docking energy,
- Applying the equal-width technique, which subdivides the variable into a fixed number of intervals, such that each of these has the same numerical width,
- Applying the equal-frequency technique, which subdivides the variable into a fixed number of intervals, such that each of these has the same number of molecules.

The Fayyad-Irani technique automatically identifies the optimal number of subdivision for each variable as a function of the docking energy; for the two remaining methods the default value for the number of intervals is set equal to 10.

The discretization operated on set 603A is repeated, applying the same subdivision in intervals, on set 603B, such to obtain two homogeneous data sets.

Output data

Set 606A is the discretized equivalent of set 603A, i.e. it contains the same number of molecules and all columns are of nominal type.

Set 606B is the discretized equivalent of set 603B, i.e. it contains the same number of molecules and all columns are of nominal type.

**2.2.2 Simple sampling**

Input data

Set 606A.

Function

The simple sampling executes a random, uniform selection of the molecules contained in the input dataset, generating K new datasets, k-1 to be used for the Bayesian model training, the remainder to verify the performance. Each subset has the same number of molecules, with the exception of the last that also incorporates the excess in the case in which the total number of molecules is not a multiple of K.

Output data

Sets 607A1, 607A2, ..., 607AK which do not have modifications from the columns standpoint (variables), but which are a subdivision of the input set molecules into K subsets.

**2.2.3 Naïve Bayes construction**

Input data

Set 607A1, set 607A2, ..., set 607AK.

Function

This operation produces a Naïve Bayes type model. The model structure links the variable class, docking energy, with all the remaining variables of the input dataset obtained through the operation of discretization. In other words there exists an oriented arc connecting the docking energy node (departure node) with each output variable of the discretization operation.

Within the K dataset, input k-1 are utilised in the model's training step, the remainder is utilised instead for an out of sample valuation of the constructed model.

Output data

Sets 608A1, 608A2, ..., 608AK are equal to the respective input sets (same molecules and characterisation variables), with the exception of the addition of the forecast column of the docking energy computed by the model.

The NB model contains the graphic structure and quantitative valorisation of the constructed classifier.

**2.2.4 Tree-Augmented Naïve Bayes construction**

Input data

Set 607A1, set 607A2, ..., set 607AK.

Function

This operation produces a Tree-Augmented Naïve Bayes type model. The model structure links, as for the Naïve Bayes model, the variable class, the docking energy, with all remaining variables of the input dataset obtained through the discretization operation. These may not be independent from each other, with the limitation that each variable may have at the most one other father in addition to the docking energy. Thus each variable of molecule characterisation, deriving from the discretization operation, has an arc coming from the docking energy and may have another incoming arc whose origin gives another characterisation variable.

Within the K dataset, input k-1 are utilised in the model's training step, the remainder is instead utilised for an out of sample valuation of the constructed model.

Output data

Set 609A1, 609A2, ..., 609AK are equal to respective input sets (same molecules and characterisation variables), with the exception of the addition of the column of the forecast docking energy computed by the model.

The TAN model contains graphic structure and quantitative valorisation of the constructed classifier.

**2.2.5 Cross Validation evaluation**

Input data

Set 608A1, set 608A2, ..., set 608AK when a Naïve Bayes model is constructed.

Set 609A1, set 609A2, ..., set 609AK when a Tree-Augmented Naïve Bayes type model is generated.

Function

This operation guides the cross validation step and produces the summary statistics on the obtainable degree of precision with the class of models known in the art with the term Naïve Bayes and Tree-Augmented Naïve Bayes.

```
                    valore reale
                ------------------
  v  p        |        |        |
  a  r        | t_pos  | f_pos  | pos_p
  l  e        |        |        |
  o  v         ------------------
  r  i        |        |        |
  e  s        | f_neg  | t_neg  | neg_p
     t        |        |        |
     o         ------------------

                 pos      neg
```

| Accuracy = (t_pos + t_neg) / (pos+neg) | |
|---|---|
| classe pos | classe neg |

Table continued

| Accuracy = (t_pos + t_neg) / (pos+neg) | |
|---|---|
| - true rate = t_pos / pos_p | - true rate = t_neg / neg_p |
| - false rate = f_neg / neg_p | - false rate = f_pos / pos_p |
| - performance = t_pos / pos | - performance = t_neg / neg |

The preceding table is related to a docking energy discretized into two classes (default), the presence of more classes is a natural extension of the preceding table.

The real value corresponds with the docking energy calculated with the simulation process, the predicted value is the docking energy classified with the aid of the Bayesian model.

In the set of iterations, the preceding measures (accuracy, true rate, false rate and performance by class) are given as average samples.

Output data

Performance data of the NB model, when a classifier of Naïve Bayes type is trained.

Performance data of the TAN model, when a classifier of Tree-Augmented Naïve Bayes is trained.

Performance data is furnished by way of contingency tables and average sample measures.

**3) Choice of model**

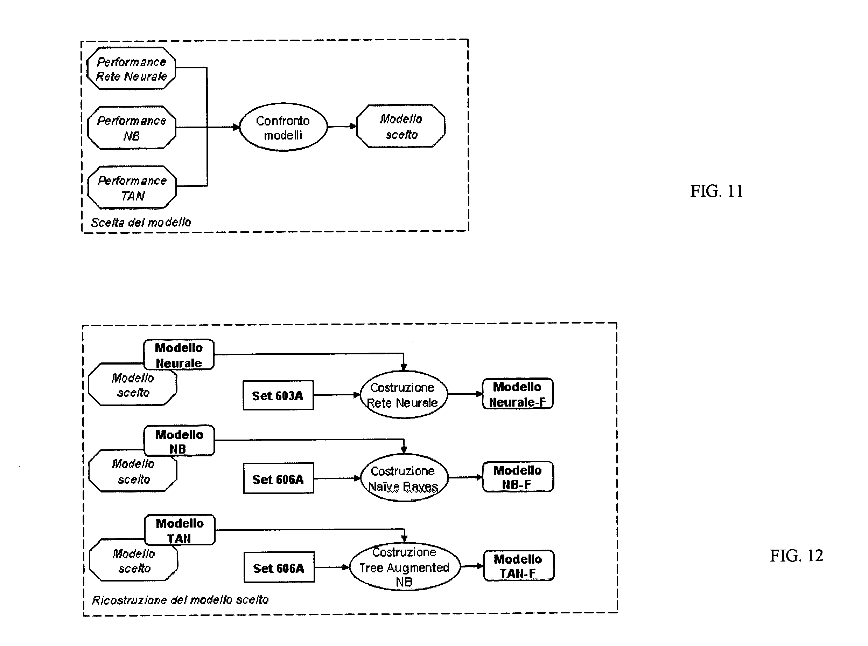

Figure 11 shows the structure of the substep of model choice with the best forecast/classification characteristics.

**3.1 Model comparison**

Input data

Neural rate performance, NB performance, TAN performance.

Function

In this operation the performances of the neural, Naïve Bayes and Tree-Augmented Naïve Bayes models are compared in order to identify which model generates the lowest error.

The neural models and the Bayesian classifiers have different features, in that the first class of models gives a numeric forecast of the docking energy, while the second only an estimate of the class to which it belongs. The choice criterion is therefore based not only on quantitative data but also on qualitative evaluations, given the profound difference of the modelling aspect of the models in question.

The forecast/classification errors are an indication of the errors that may be committed on the group B molecules.

Output data

Chosen model to be used for estimating the docking energy of the group B molecules.

**4) Reconstruction of the chosen model**

Figure 12 shows the structure of the substep of chosen model reconstruction, utilising all of the molecules of group A.

The model reconstruction is dependent on the evaluation carried out in the preceding step, in particular:

Neural Model. Set 603A is utilised for the generation of the Neural-F Model, which has the same architecture of the chosen Neural Model.

- NB Model. The set 606A is used for NB-F Model learning, without the application of cross validation techniques.
- TAN Model. The set 606A is used for the TAN-F Model learning, without the application of cross validation techniques.

The reconstruction of the model is carried out for taking advantage of all the available information in the group A molecules; in the performance determination and choice step, in fact, a part of it is used as an out of sample evaluation set.

## 5) Obtaining the docking energy

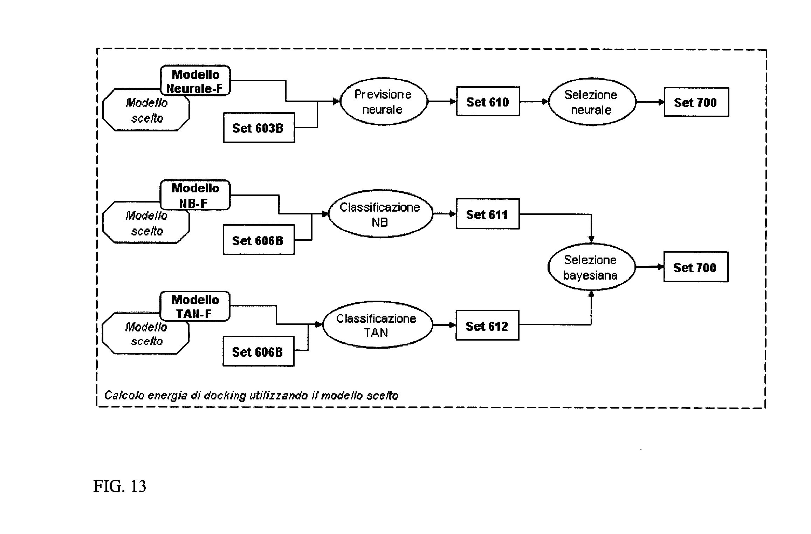

Figure 13 shows the structure of the docking energy obtainment substep of the group B molecules.

This substep is dependent on the generated and chosen model, whether a neural network or a Bayesian classifier. In both cases, after the forecast/classification operation is executed, the choice of the molecules that will make up the set 700 is carried out, from which the actual docking energy will be obtained with a simulation process.

The estimation of the errors that may be committed in forecast/classification is given by the actual performances of the chosen model.

### 5.1 Neural forecast

Input data

Neural-F model, set 603B.

Function

In this operation the docking energies of the group B molecules contained in set 603B are forecasted.

The computation utilises the Neural-F model, built in the preceding substep, which contains the qualitative/ quantitative valorisation of the acquired knowledge.

Output data

Set 610, equal to input set 603B, with the addition of the docking energy forecast.

### 5.2 Neural selection

Input data

Set 610.

Function

In this operation the molecules of set 610 are chosen which present a docking energy less than or equal to a certain threshold. Such threshold is by default set at -6.5 + the average error of forecast of the neural network for values less than -6.5 (normally equal or greater than -6).

Output data

Set 700, which contains the candidate molecules of the simulation processes and subsequent optimisations.

### 5.3 NB classification

Input data

NB-F model, set 606B.

Function

In this operation the docking energies of the group B molecules contained in set 606B are classified.

The computation uses the NB-F model, built in the preceding substeps, which contains the qualitative/quantitative valorisation of the acquired knowledge.

Output data

Set 611, equal to the input set 606B, with the addition of the docking energy classification.

**5.4 TAN classification**

Input data

TAN-F model, set 606B.

Function

In this operation the docking energies of the group B molecules contained in set 606B are classified.
The computation uses the TAN-F model, built in the preceding substeps, which contains the qualitative/quantitative valorisation of the acquired knowledge.

Output data

Set 612, to the input set 606B, with the addition of the docking energy classification.

**5.5 Bayesian selection**

Input data

Set 611 in the case of use of the Naive Bayes model, set 612 when the Tree-Augmented Naïve Bayes model is used.

Function

In this operation the molecules are chosen from the set 611 or 612 which have a docking energy classification in the class or in the classes which identify values less than or equal to a certain threshold (in the case of the two classes chosen by default in the discretization step, the class which corresponds to docking energy values less than or equal to -6.5).

Output data

Set 700, which contains the candidate molecules for the simulation processes and subsequent optimisations.

*Multiple Objective Optimisation Conducted with Genetic Algorithms*

The number of compounds included in the second library 400, denoted $C_i$, is equal to $n$: { $C_i$ $i$=1,...$n$, $|C|$=$n$}; as seen each compound is characterised by the specific values of a number of descriptors, equal to $n_d$, which also coincides with the dimensionality of the entire space of parameters. The descriptors are indicated as $D_{ji}$ ($j$=1,...$n_d$, $i$=1,...$n$). In the context of the description of this further step 9 of the method of the invention, even the docking energy calculated by direct method (step 7) or evaluated using the classifier (step 8) is understood to be one of the molecular descriptors. The problem to be confronted consists for example in the identification among all $D_j$ of a "pivot" descriptor to use as ranking parameter of the C elements. The docking energy of the actual compound within the considered receptor site (or its experimental equivalent, binding constant) is often referred to as pivotal element. In this manner in fact the optimal subset 700 was calculated in step 8 of the invention. In many cases, nevertheless, the simple ordering of the $C_i$ elements according to increasing values of one single pivot parameter (such as the docking energy) is inadequate, because one single parameter is not generally sufficient to define in a global manner the compound's fitness. The true clarification of the library 400 consists in the identification of the compound group $C_i$ which simultaneously maximises more conditions (or possesses more characteristics in the maximum level). More in particular, the following cases are considered which are indicative of situations often encountered in the practice of virtual library structuring and optimisation:

1- Identify a compound group $C_i$ which has maximum docking energy and *simultaneously* the maximum similarity (or dissimilarity) within a subgroup with respect to a subset of $D_j$.

2- Identify a compound group $C_i$ which has maximum docking energy and is maximally similar to the "best compounds" of all $C_i$ with respect to one or more $D_j$ characteristics.

3- Identify a group $C_i$ which has maximum docking energy and shares specific pharmacokinetics characteristics with other molecules (outside library C).

There have only been examples that include the condition of maximum docking energy, keeping in mind the hypothesis that the second library under examination is geared to the discovery of a small group of lead pharmacological compounds. Of course the list of examples may be even more broken down in different functionality examinations different from those of the lead compounds. One strategy for obtaining this result may lead to the specialisation of the library 400 through an iterative process of optimisation which explores the combinatory library by way of subsequent cycles of compound selection, modification and testing for their pharmacological properties and activity levels. In this step lead compounds are understood to be a set of sample molecules with good properties of pharmacological activity.

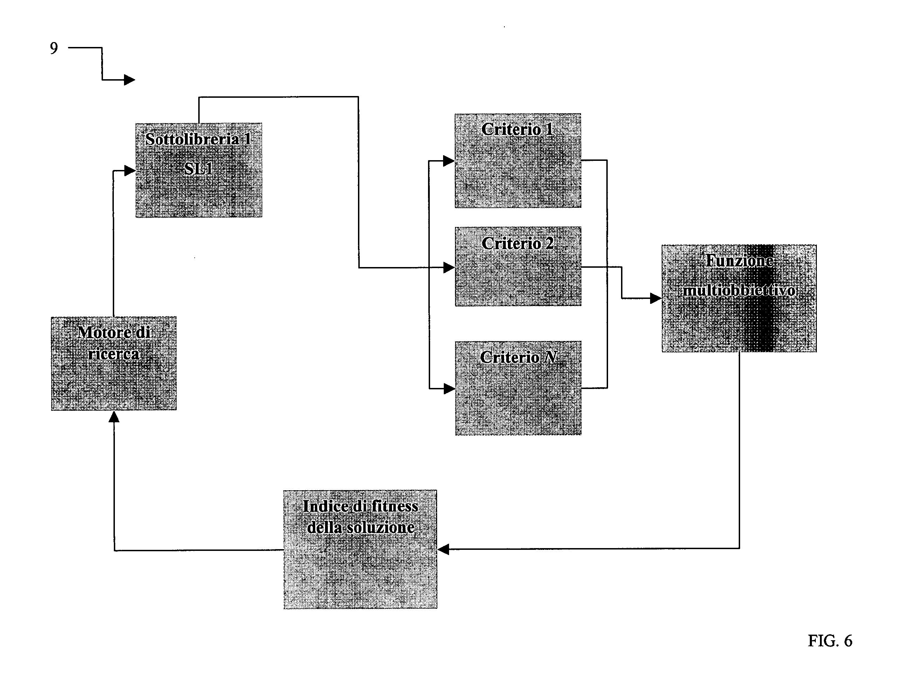

The general architecture of the iterative scheme is represented in Fig. 6.

The "Search Engine" substep may be based on different algorithms: in the invention method, "accelerated" genetic algorithms are preferably employed through MCMC *(Markov Chain Monte Carlo)* techniques. One advantage of this approach is that the search engine, formulated as optimiser, with a sufficiently general and flexible solution algorithm, may comprise different performance measures. The solution proposed by the optimisation algorithm in a generic iteration will be indicated with the term "state" (design).

The number of possible states, presuming the desire to identify 50 compounds in a library of 1000, is

$$\binom{1000}{50} = 10^{85}$$

, which implies how the use of approximation methods is absolutely necessary. The criteria that will be utilised in this step 9 for guiding the search and optimisation engine are listed here below.

A set of K compounds is given along with a set of lead compounds $l$; $d_{il}$ is the distance between the i compounds and the lead $l$ in a space of molecular descriptors; f the Kernal function (for default identity). Two important indices whose trade-off guarantees an equilibrated library are:

**i) *Similarity***

The similarity index, (to minimise) is given by:

$$S(s) = \frac{1}{k} \sum_{i=1}^{k} f[\min_{l}(d_{il})]$$

This index compares the elements i of a set of compounds s with the elements $l$ of a set of lead compounds; the smaller the distance between the elements and lead compounds, the better the library.

ii) Diversity

The diversity index (to maximise) is given by:

$$D(s) = \frac{1}{k} \sum_{a} f[\min_{b \neq a}(d_{ab})]$$

This index instead compares elements within one set of compounds, measuring the reciprocal diversity. The diversity between the elements of a library is fundamental in order to avoid the creation of a set of molecules characterised by an overly emphasised similarity with a single lead compound.

One computationally efficient implementation of the calculation of S and D organises all admissible states (designs) in a k-dimensional tree in order to subsequently carry out a *"nearest neighbourhood search"* for each compound.

**iii) *Complementarity***

This criterion is closely connected to the diversity and indicates the capacity of a set of S* states to "add" diversity to a pre-existing library.

$$D(S, S^*) = D(S \cup S^*) = \frac{1}{k} \sum_{i} f[\min_{j \neq i}(d_{ij})]$$

where i and j are the indices of the compounds in S and S*, while $k=|S|+|S^*|$. This index is closely correlated to the *Diversity* and stands out only for the fact that intrinsic diversity is not the only type of diversity to be considered.

### iv) *Confinement*

This criterion measures the degree with which the properties of a given set of compounds fall within established limits:

$$P(S) = \frac{1}{k}\sum_i \sum_j \max(x_j^{min} - x_{ji}, x_{ji} - x_j^{max}, 0)$$

where $x_{ji}$ indicates the j property of the compound i and x $x_j^{min}$, , $x_j^{max}$ indicate the range of admissible values.

When the important property must reach a specific "target" value $x_j^*$, then it is possible to return to the definition

$$P(S) = \frac{1}{k}\sum_i \sum_j |x_{ij} - x_j^*|.$$

### v) *Distribution*

This criterion is utilised for the selection of states (designs) that have a predefined molecular distribution.

$$K^* = \max |P(x) - P^*(x)| \text{ (Criterion of Kolmogorov Smirnov)}$$

where $P(x)$ is an estimator of the empirical distribution and $P^*$ is the target distribution.

### vi) *Activity*

One fundamental objective in the design of a combinatorial library consists in the selection of the most active compounds, which may be obtained by considering the "average predicted activity" index

$$Q_a = \frac{1}{k}\sum a_i$$

where $a_i$ is a measure of the prestated activity of the compound i. By maximising $Q_a$ it is possible to obtain libraries that are better targeted toward specific objects.

### vii) *Selectivity*

Indicates the selectivity relative to a set of biological targets:

$$Q_s = \frac{1}{k}\sum_i [a_{iq} - \max_{j \neq q}(a_{ij})]$$

where $a_{ij}$ is the predicted activity of the compound with respect to the target *j* and q is the target for which the molecules must be selective.

Complex objective functions that encompass diverse indices have many minima and require a stochastic approach capable, with a higher probability, of identifying the global minimum. One generalisation of the optimisation method with genetic algorithms, strictly connected to BOA, is the "Simulated Annealing" in the Metropolis Monte Carlo (MMC) version. One state is defined as a particular choice of compounds (a list of subsets from one or more virtual libraries) and step of the MMC algorithm is given by a "little" change of state (i.e. the substitution of a small percentage of compounds with others outside the state). The objective function associates a fitness index with each state. A state transition is accepted if it leads the system to an improved level of fitness, otherwise it is accepted with a probability that depends from the fitness difference between the two states. A new optimal subset 700' of the second library of compounds 400 is obtained in this manner, which simultaneously maximises more conditions (or possesses more characteristics in the maximum level).

**Claims**

1. Method of construction and selection of virtual libraries in combinatorial chemistry, comprising the steps of:

   - Providing a three-dimensional structure of a target macromolecule (PROT);
   - Determining one or more receptor sites of said target macromolecule (PROT);
   - Creating a first virtual library (300) of compounds deriving from at least one input library (100;200);
   - Calculating a plurality of molecular descriptors for each molecule of said virtual library (300), generating a second virtual library (400) containing each molecule of said first library (100; 200; 50) and values of said molecular descriptors;
   - Selecting a representative subset (500) of said second virtual library (400);
   - Calculating for each molecule belonging to said representative subset a value of a quantity ($E_{dock}$) associated with the formation of a bond between said target macromolecule (PROT) and said molecule belonging to said representative subset (500);
   - Obtaining by way of simulation through "Machine Learning" methods for each molecule of a plurality of molecules of said second virtual library (400) and not belonging to said representative subset (500) a value of said quantity ($E_{dock}$) associated with the formation of a bond between said target macromolecule (PROT) and said each molecule of a plurality of molecules not belonging to said representative subset (500).

2. Method according to claim 1, comprising the step of obtaining an optimal subset (700, 700') of said second virtual library (400) as a function of the value of said quantity ($E_{dock}$) associated with the formation of a bond between said target macromolecule and said molecule of said second virtual library (400).

3. Method according to claim 1 or 2, in which said simulation of the value of said quantity ($E_{dock}$) is obtained through the use of neural networks.

4. Method according to claim 1 or 2, in which said simulation of the value of said quantity ($E_{dock}$) is obtained through the use of Bayesian classifiers.

5. Method according to one or more of the preceding claims, in which said step of creating a first virtual library (300) of compounds deriving from at least one input library (100; 200), comprises the substeps of:

   - Providing a first input library (100; 50) containing a plurality of scaffolds ($S_i$);
   - Providing a second input library (200; 50) containing a plurality of substituents ($R_i$);
   - For each scaffold ($S_i$) of said first library (100), generating a plurality of molecules obtained through bonding between said scaffold ($S_i$) and each of the substituents ($R_i$) of said second input library (200);
   - Repeating the preceding step for all scaffolds ($S_i$) of said first input library (100).

6. Method according to claim 5, comprising the steps of

   - Identifying a plurality of connection points for said substituents ($R_i$) for each scaffold ($S_i$) of said first library;
   - Bonding said substituents ($R_i$) to each connection point of said scaffold ($S_i$) for each scaffold of said first library (100).

7. Method according to claim 5 or 6, comprising the step of orienting said substituents ($R_i$) in the bond with said scaffold ($S_i$) so that a molecule having approximately the minimum global energy is obtained.

8. Method according to one or more of the preceding claims, comprising the step of

   - Calculating the three-dimensional structure of each of the molecules of said first virtual library (300);
   - Optimising said three-dimensional structure using approximate quantum mechanical methods.

9. Method according to one or more of the preceding claims, in which said representative subset (500) is determined by selecting the molecules of said second virtual library (400) having the greatest dissimilarity between each other.

**10.** Method according to one or more of the preceding claims, in which said quantity associated with the formation of a bond between said target macromolecule (PROT) and a molecule of said second virtual library (400) is the docking energy ($E_{dock}$).

**11.** Method according to one or more of the claims from 3 to 9, comprising the step of generating a training set (600) for training said methods of "Machine Learning", said training set (600) comprising said molecules belonging to said representative subset (500), said molecular descriptors of said molecules of said representative subset and said quantities ($E_{dock}$) calculated for said molecules of said representative subset (500).

**12.** Method according to claim 11, in which the step of subdividing said training set (600) into K subsets is foreseen, K-1 of which are used for building a forecast/classification model and the remaining subsets are used as test set.

**13.** Method according to claims 11 or 12, in which said step of obtaining by way of simulation through Machine Learning methods the value of said quantity ($E_{dock}$) associated with the formation of a bond between said target macromolecule and said each molecule of a plurality of molecules not belonging to said representative subset comprises the substeps of:

- Constructing a neural network model;
- Constructing a Bayesian classifier of Naive Bayes type;
- Constructing a Bayesian classifier of Tree-Augmented Naive Baies type;
- Comparing the performance of said models, using as input data at least a fraction of said training set (600);
- Selecting the model having the least error in order to estimate said quantity ($E_{dock}$).

**14.** Method according to one or more of the preceding claims comprising the steps of:

- Selecting several of said descriptors as pivot descriptors;
- Generating an optimal subset (700') of said second virtual library (400) as a function of the value of said quantity ($E_{dock}$) associated with the formation of a bond between said target macromolecule and said molecule of said second virtual library and the value of said pivot descriptors.

**15.** Method according to one or more of the preceding claims, in which said target macromolecule (PROT) is a protein.

TABLE 1 -

| LIST OF MOLECULAR DESCRIPTORS | |
|---|---|
| 1 EDock | Docking Energy |
| 2-15 CHGR_SAS# | Surface area accessible to the solvent summed on atoms with charge within the following intervals. The # symbol may at present be equal to m0,.....m6 or p0,... p6. An interval is associated with each # case, as follows: m0=[-0.05,0.00) m1=[-0.10,-0.05) m2=[-0.15,-0.10) m3=[-0.20,-0.15) m4=[-0.25,-0.20) m5=[-0.3,-0.25) m6= $q_i$ <- 0.3 p0=[0.00,0.05) p1= [0.05,0.10) p2=[0.10,0.15) p3=[0.15,0.20) p4=[0.2,0.25) p5=[0.25,0.3) p6= $q_i$ > 0.3 |
| 16-29 CHGR_VOL# | Summed atomic volumes on atoms with charge within the # intervals (see 2-15) |
| 30-43 CHGR_VSA# | Summed Van der Waals atomic surfaces on atoms with charge within the # intervals (see 2-15) |
| 44 -47 CHGR_pmi# | Sum of the principal weighted moments with the atomic charge, where #=null, #=X x component, #=Y y component #=Z z component |
| 48 Fcharge | Total[76]charge |
| 49 Heatform | Heat of formation |
| 50 Kier1 | First index k $=A(A-1)^2/B^2$ where A=number of atoms, B=number of cancelled bonds in the graph |
| 51 Kier2 | Second index k$=(A-1)(A-2)^2/{}^2P^2$ $^2P$ is the path of length 2 of the graph |
| 52 Kier3 | Third index k $(A-3)(A-2)^2/{}^3P^2$ with even A's $(A-1)(A-3)^2/{}^3P^2$ with odd A's |

Table continued

| LIST OF MOLECULAR DESCRIPTORS | |
|---|---|
| 1 EDock | Docking Energy |
| 53 KierA1 | First modified index $\alpha$ $(A+a)(A+a-1)^2/(B+a)^2$ where $a=r_i/(r_c-1)$ $r_i$ is the covalent radius of the i[esimo] atom $r_c$ is the covalent radius of the carbon atom |
| 54 KierA2 | Second modified index $\alpha$ $(A+a-1)(A+a-2)^2/(^2P+a)^2$ |
| 55 KierA3 | Third modified index $\alpha$ $(A+a-3)(A+a-2)^2/(^3P+a)^2$ A's even $(A+a-1)(A+a-3)^2/(^3P+a)^2$ A's odd |
| 56 KierFlex | (KierA1)(KierA2)/A |
| 57-60 MASS_pmi# | Sum of the principal inertia moments, #=null, #= X x component, #=Y y component, #=Z z component |
| 61 -64 NULL_pmi# | Sum of the principal non-weighted moments, #=null, #= X x component, #=Y y component, #=Z z component |
| 65 -68 POLA_pmi # | Sum of the principal weighted moments with the atomic polarisability, #=null, #= X x component, #=Y y component, #=Z z component |
| 69-71 QM_dip# | Sum of the dipole moments, #= X x component, #=Y y component, #=Z z component |
| 72 QM_dipt | Tota[77]dipole moment |
| 73 Q_PCm | Total negative charge |
| 74 Q_PCp | Total positive charge |
| 75 Q_RPCm | Maximum relative negative partial charge (qi>0)/Q_PCm |
| 76 Q_RPCp | Maximum relative positive partial charge (qi<0)/Q_PCp |
| 77-82 Q_SAS_F# | Total SASA fraction of # atoms # is hydrophilic hydrophobic negative polar positive weakly polar |
| 83-88 Q_SAS_# | Total SASA of # atoms, where # is (77-82) |
| 89-94 Q_VOL_F# | Total fraction of atomic volume of # atoms, where # is (77-82) |
| 95-100 Q_VOL_# | Total atomic volume of # atoms, where # is (77-82) |
| 101-106 Q_VSA_F# | Total VSA fraction of # atoms, where # is (77-82) |
| 107-112 Q_VSA_# | Total VSA sum of # atoms, where # is (77-82) |
| 113 R_pcpm | Distance between Q_PCp and Q_PCm |
| 114 -117 SAND_pmi# | Sum of principal weighted moments with Sanderson electronegativity #=null, #=X x component #=Y y component #=Z z component |
| 118 SASA | Surface area accessible to the solvent |
| 119 -126 SASA_# | SASA of # atoms, where # are Hydrogen bond acceptors Acidic atoms Hydrogen bond donors Hydrophilic atoms Hydrophobic atoms Polar atoms Weakly polar atoms |
| 127-134 SMR_SAS# | Surface area accessible to the summed solvent on the atoms with a molecular refractivity within the limits of #, where # is SAS0 [0, 1.14] SAS1 (1.14,2.28] SAS2 (2.28,3.42] SAS3 (3.42,4.56] SAS4 (4.56,5.70] SAS5 (5.70, 6.84] SAS6 (6.84,7.98 ] SAS7 ar_i>7.98 |
| 135-142 SMR_VOL# | Summed atomic volume of atoms with molecular refractivity within the limits of #, where # is (127-134) |
| 143-150 SMR_VSA0 | Summed VSA on the atoms with molecular refractivity within the limits of #, where # is (127-134) |
| 151 SlogP | Logarithm of the partition coefficient P= octanol/water |

Table continued

| LIST OF MOLECULAR DESCRIPTORS | |
|---|---|
| 1 EDock | Docking Energy |
| 152 - 161 SlogP_SAS# | Summed SASA of atoms with SlogP within the limits of #, where # is, SAS0 SlogP < -0.4 SAS1[79]SlogP (-0.4, -0.2] SAS2 SlogP (-0.2, 0] SAS3 SlogP (0, 0.1] SAS4 SlogP (0.1, 0.15] SAS5 SlogP (0.15, 0.2] SAS6 SlogP (0.2, 0.25] SAS7 SlogP (0.25,0.3] SAS8 SlogP (0.3, 0.35] SAS9 SlogP>0.35 |
| 162 -171 SlogP _VOL# | Summed atomic volume on atoms with SlogP in the # interval (see 162-171) |
| 172 -181 SlogP_VSA# | Summed VSA on atoms with SlogP in the # interval (see 162-171) |
| 182 VAdjEq | $1+\log_2 m$ where m=heavy/(heavy bonds) if m>0, if m=0 VadjMA=0 |
| 183 VAdjMa | $-(1-f)\log_2(1-f)-f\log_2 f$ where $f =(n^2-m)/ n^2$ n is the number of heavy atoms, m is the number of bonds between heavy atoms. If f is not within the limits (0,1), Vadj Eq=0 |
| 184-187 VDWV_pmi# | Sum of principal weighted moments with volumes VdW # =zero, #=X x component, #=Y y component, #=Z z component |
| 162 -171 SlogP_VOL# | Summed atomic volume of atoms with SlogP in the # interval (see 162-171) |
| 188 -195 VOL_# | Atomic volumes of # atoms, where # are the Hydrogen bond acceptors Acidic atoms Hydrogen bond donors Hydrophilic atoms Hydrophobic atoms Polar atoms Weakly polar atoms |
| 197 -203 VSA_# | VSA of # atoms, where # is see 188-195 |
| 204 Weight | Molecular weight |
| 205 X_pcminus | X component of the negative charge centre |
| 206 X_pcplus | X component of the positive charge centre |
| 207 Y_pcminus | Y component of the negative charge centre |
| 208 Y_pcplus | Y component of the positive charge centre |
| 209 Z_pcminus | Z component of the negative charge centre |
| 210 Z_pcplus | Z component of the positive charge centre |
| 211-238 a_C# | Carbon atoms with ccg codes between 601 and 628 |
| 239 a_IC | $\Sigma_1\beta_i\log\beta_1$ where $\beta_i=n_i/n$ $n_i$ is the number of atoms with atomic number $Z_i$ |
| 240 a_ICM | a_IC*n |
| 241 -255 a_N# | Nitrogen atoms with ccg codes between 701 and 715 |
| 256 -268 a_O# | Oxygen atoms with ccg codes between 801 and 813 |
| 269 a_S# | Sulphur atoms with ccg codes between 1601 and 1603 |
| 272 -275 a_# | # Atoms<br>Where # is<br>Hydrogen bond donors<br>Acidic atoms<br>Aromatic atoms<br>Basic atoms |
| 276 a_count | Total number of atoms |
| 277-280 a_# | # Atoms<br>Where # is<br>Hydrogen bond donors<br>Hydrophilic atoms<br>Hydrophobic atoms<br>Heavy atoms |

Table continued

| LIST OF MOLECULAR DESCRIPTORS | |
|---|---|
| 1 EDock | Docking Energy |
| 281 a_nB | Number of Boron atoms |
| 282 a_nBR | Number of Bromine atoms |
| | |
| 283 a_nC | Number of Carbon atoms |
| 284 a_nCL | Number of Chlorine atoms |
| 285 a_nF | Number of Fluorine atoms |
| 286 a_nH | Number of Hydrogen atoms |
| 287 a_nI | Number of Iodine atoms |
| 288 a_nN | Number of Nitrogen atoms |
| 289 a_nO | Number of Oxygen atoms |
| 290 a_nP | Number of Phosphorus atoms |
| 291 a_nS | Number of Sulphur atoms |
| 292 amide | Amide |
| 293 amine | Amine |
| 294 apol | Sum of atomic polarisabilities |
| 295 b_1rotR | Number of single rotating bonds |
| 296 b_ar | Number of aromatic bonds |
| 297 b_count | Total number of bonds (including H) |
| 298 b_double | Number of double bonds |
| 299 b_heavy | Number of heavy bonds |
| 300 b_single | Number of single bonds |
| 301 b-triple | Number of triple bonds |
| 302 chi0 | Atomic connectivity of order 0 $\Sigma 1/sqrt(d_i)$ |
| 303 chi0_C | Atomic connectivity of the carbon of order 0 $E1/sqrt(d_i)$ |
| 304 chi0v | Atomic connectivity of order 0 $\Sigma 1/sqrt(d_i)$ calculated on heavy atoms |
| 305 chi0v_C | Atomic connectivity index of valence of order 0 $\Sigma 1/sqrt(v_i)$ calculated on carbon atoms with $v_i>0$ |
| 306 chi1 | Atomic connectivity index of order 1 $=\Sigma 1/sqrt(d_i d_j)$ calculated on bonds between heavy atoms i and j with i<j |
| 307 chi1_C | Atomic connectivity index of order 1 $=\Sigma, 1/sqrt(d_i dj)$ calculated on bonds between carbon atoms i and j with i<j |
| 308 chi1v | $=\Sigma, 1/sqrt(v_1 vj)$ calculated on bonds between heavy atoms i and j with i<j |
| 309 chi1v_C | Atomic connectivity index of valence of order 1 $=\Sigma, 1/sqrt(v_1 v_j)$ calculated on the bonds between carbon atoms with i<j |
| 310 density | Density |
| 311-313 dipX_co# | Dipole moment #=X x component, #=Y y component #=Z z component |
| 314 dipt_co | Total dipole moment |

Table continued

| LIST OF MOLECULAR DESCRIPTORS | |
|---|---|
| 1 EDock | Docking Energy |
| 315 -317 fCHGR_pmi# | Principal fractional weighted moment with the atomic charges #= X x component, #=Y y component #=Z z component |
| 318-320 fMASS_pmi# | Principal fractional weighted moment with the atomic masses #= X x component, #=Y y component #=Z z component |
| 321-323 fNULL_pmi# | Principal fractional moment #= X x component, #=Y y component #=Z z component |
| 324-326 fPOLA_pmi# | Principal fractional weighted moment with the polarisability moment #= X x component, #=Y y component #=Z z component |
| 327 -329 fSAND_pmi# | Principal fractional weighted moment with Sanderson electronegativity #= X x component, #=Y y component #=Z z component |
| 330-332 fVDWV_pmi# | Principal fractional weighted moment with the VdW volume #= X x component, #=Y y component #=Z z component |
| 333 glob | Principal moment x component/z component |
| 334 logS | Solubility logarithm |
| 335 mr | Molecular refractivity |
| 336 pioniz | Ionisation potential |
| 337 quadXX_co | Moment of quadrupole xx component |
| 338 quadXY_co | Moment of quadrupole xy component |
| 339 quadXZ_co | Moment of quadrupole xz component |
| 340 quadYY_co | Moment of quadrupole yy component |
| 341 quadYZ_co | Moment of quadrupole yz component |
| 342 quadZZ_co | Moment of quadrupole zz component |
| 343 quadt_co | Quadrupole moment |
| 344 rgyr | Gyration radius |
| 345 vdw_area | Van der Waals surface calculated with a connection table |
| 346 vdw_vol | Van der Waals volume calculated with a connection table |
| 347 zagreb | $\Sigma_i d_i^2$ |

FIG. 1

$X_1 \text{----} O$

$X_2 \text{----} S$

$X_3 \text{----} C=O$

$X_4 \text{----} C=S$

$X_5 \text{----} NH$

$S_2, A_2 = 3$

$S_4, A_4 = 5$

$S_1, A_1 = 2$

$S_3, A_3 = 4$

$R_1 = H$   $R_2 = CH_3$   $R_3 = C_2H_5$   $R_4 = OH$   $R_5 = OCH_3$   $R_6 = OC_2H_5$

$R_7 = SH$   $R_8 = SCH_3$   $R_9 = SC_2H_5$   $R_{10} = NH_2$   $R_{11} = HNCH_3$   $R_{12} = HNC_2H_5$

$R_{13} = N(CH_3)_2$   $R_{14} = N(C_2H_5)_2$

FIG. 2

36

FIG. 3

7

Definizione dei valori massimi per $t_x,t_y,t_z$, sulla base delle dimensioni della griglia: $T_x, T_y, T_z$.

Scelta random dei valori attuali di $t_x,t_y,t_z, s_x,s_y,s_z$ e . Costruzione della matrice di rototraslazione **T**.

Trasformazione delle coordinate cartesiane del ligando A

Calcolo dell'energia di docking utilizzando le coordinate attuali

NO

$E_{dock}$ minima?

SI

FIG. 4

38

Fig. 5

FIG. 6

Funzione
multiobbiettivo

Criterio 1

Criterio 2

Criterio N

Indice di fitness
della soluzione

Sottolibreria 1
SL1

Motore di
ricerca

9

Fig. 7

FIG. 8

FIG. 9

Costruzione modelli Naïve Bayes e Tree Augmented Naïve Bayes

FIG. 10

EP 1 628 234 A1

EP 1 628 234 A1

**FIG. 11**

Performance
Rete Neurale

Performance
NB

Performance
TAN

Confronto
modelli

Modello
scelto

Scelta del modello

**FIG. 12**

Modello
Neurale

Modello
scelto

Set 603A

Costruzione
Rete Neurale

Modello
Neurale-F

Modello
NB

Modello
scelto

Set 606A

Costruzione
Naïve Bayes

Modello
NB-F

Modello
TAN

Modello
scelto

Set 606A

Costruzione
Tree Augmented
NB

Modello
TAN-F

Ricostruzione del modello scelto

FIG. 13

**European Patent Office**

## EUROPEAN SEARCH REPORT

Application Number

EP 04 42 5416

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FLORIANO WELY B ET AL: "HierVLS hierarchical docking protocol for virtual ligand screening of large-molecule databases." JOURNAL OF MEDICINAL CHEMISTRY, vol. 47, no. 1, 1 January 2004 (2004-01-01), pages 56-71, XP002361267 ISSN: 0022-2623 * page 56, line 1 - line 28 * * page 56, left-hand column, line 29 - page 59, right-hand column, line 20 * * page 62, left-hand column, line 35 - page 64, left-hand column, line 20 *<br>----- | 1-15 | G06F19/00 |
| A | MURCIA MARTA ET AL: "Virtual screening with flexible docking and COMBINE-based models. Application to a series of factor Xa inhibitors." JOURNAL OF MEDICINAL CHEMISTRY. 12 FEB 2004, vol. 47, no. 4, 12 February 2004 (2004-02-12), pages 805-820, XP002361268 ISSN: 0022-2623 * page 805, line 1 - line 17 * * page 805, left-hand column, line 1 - page 809, right-hand column, line 5 * * page 810, left-hand column, line 10 - page 814, right-hand column, line 3 * * page 815, left-hand column, line 17 - page 818, right-hand column, line 48 *<br>-----<br>-/-- | 1-15 | TECHNICAL FIELDS SEARCHED (IPC)<br><br>G06F |

The present search report has been drawn up for all claims

| Place of search | Date of completion of the search | Examiner |
|---|---|---|
| Munich | 2 January 2006 | Barba, M |

EPO FORM 1503 03.82 (P04C01)

**European Patent Office**

# EUROPEAN SEARCH REPORT

Application Number

EP 04 42 5416

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SPROUS DENNIS G ET AL: "OptiDock: Virtual HTS of combinatorial libraries by efficient sampling of binding modes in product space"<br>JOURNAL OF COMBINATORIAL CHEMISTRY,<br>vol. 6, no. 4, 15 April 2004 (2004-04-15),<br>pages 530-539, XP002361269<br>ISSN: 1520-4766<br>* page 530, line 1 - line 15 *<br>* page 530, left-hand column, line 1 - page 533, right-hand column, line 9 *<br>* page 534, left-hand column, line 10 - page 535, right-hand column, line 25 *<br>----- | 1-15 | |
| A | US 6 240 374 B1 (CRAMER RICHARD D ET AL) 29 May 2001 (2001-05-29)<br>* column 9, line 59 - column 11, line 51 *<br>* column 13, line 53 - column 22, line 32 *<br>* column 25, line 61 - column 38, line 50 *<br>* column 38, line 59 - column 40, line 3 *<br>* column 45, line 37 - column 49, line 57 *<br>* column 56, line 19 - column 77, line 45 *<br>----- | 1-15 | TECHNICAL FIELDS SEARCHED (IPC) |
| A | WO 99/50770 A (COMBICHEM, INC; GREENE, JONATHAN, W; MOUNT, JOHN)<br>7 October 1999 (1999-10-07)<br>* page 2, line 30 - page 4, line 3 *<br>* page 4, line 30 - page 12, line 21 *<br>* page 16, line 7 - page 21, line 33 *<br>----- | 1-15 | |
| A | US 2002/138204 A1 (AFSHAR MOHAMMAD ET AL) 26 September 2002 (2002-09-26)<br>* paragraph [0014] - paragraph [0023] *<br>* paragraph [0029] - paragraph [0073] *<br>----- | 1-15 | |

The present search report has been drawn up for all claims

| Place of search | Date of completion of the search | Examiner |
|---|---|---|
| Munich | 2 January 2006 | Barba, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 42 5416

This annex lists the patent family members relating to the patent documents cited in the above-mentioned European search report.
The members are as contained in the European Patent Office EDP file on
The European Patent Office is in no way liable for these particulars which are merely given for the purpose of information.

02-01-2006

| Patent document cited in search report | | Publication date | Patent family member(s) | | Publication date |
|---|---|---|---|---|---|
| US 6240374 | B1 | 29-05-2001 | AU 1847997 A<br>CA 2245935 A1<br>EP 0892963 A1<br>WO 9727559 A1 | | 20-08-1997<br>31-07-1997<br>27-01-1999<br>31-07-1997 |
| WO 9950770 | A | 07-10-1999 | AU 3116199 A<br>BR 9909179 A<br>CA 2326134 A1<br>EP 1066578 A1<br>JP 2004515447 T<br>NO 20004831 A<br>PL 343324 A1 | | 18-10-1999<br>16-10-2001<br>07-10-1999<br>10-01-2001<br>27-05-2004<br>20-11-2000<br>13-08-2001 |
| US 2002138204 | A1 | 26-09-2002 | US 2002045168 A1 | | 18-04-2002 |

EPO FORM P0459

For more details about this annex : see Official Journal of the European Patent Office, No. 12/82